# EUROPEAN PATENT SPECIFICATION

(11) **EP 2 717 940 B1**
(45) Date of publication and mention of the grant of the patent: **22.04.2020**
(21) Application number: 12727914.9
(22) Date of filing: 13.06.2012
(51) Int. Cl.: A61M 1/36, B01J 20/32

(54) **TREATING CONDITIONS ASSOCIATED WITH METABOLIC SYNDROME**
BEHANDLUNG VON LEIDEN IN ZUSAMMENHANG MIT DEM STOFFWECHSELSYNDROM
TRAITEMENT D'ÉTATS ASSOCIÉS À UN SYNDROME MÉTABOLIQUE

(30) Priority: 13.06.2011 US 201161496167 P
(43) Date of publication of application: 16.04.2014
(73) Proprietor: TLA Targeted Immunotherapies AB, 171 76 Stockholm (SE)
(72) Inventor: COTTON, Graham, Edinburgh EH13 9PH (GB); WINQVIST, Ola, S-756 53 Uppsala (SE)
(74) Representative: Zacco Denmark A/S
(86) International application number: PCT/GB2012/051349
(87) International publication number: WO 2013/041833

(56) References cited:
- EP-A1- 2 067 495
- WO-A2-2010/029317
- R CANCELLO ET AL: "Review article: Is obesity an inflammatory illness? Role of low-grade inflammation and macrophage infiltration in human white adipose tissue", BJOG: AN INTERNATIONAL JOURNAL OF OBSTETRICS AND GYNAECOLOGY, vol. 113, no. 10, 1 October 2006 (2006-10-01), pages 1141-1147, XP055038252, ISSN: 1470-0328, DOI: 10.1111/j.1471-0528.2006.01004.x
- MAURY E ET AL: "Adipokine dysregulation, adipose tissue inflammation and metabolic syndrome", MOLECULAR AND CELLULAR ENDOCRINOLOGY, ELSEVIER IRELAND LTD, IE, vol. 314, no. 1, 15 January 2010 (2010-01-15), pages 1-16, XP026709062, ISSN: 0303-7207, DOI: 10.1016/J.MCE.2009.07.031 [retrieved on 2009-08-12]
- LUMENG CAREY N ET AL: "Obesity induces a phenotypic switch in adipose tissue macrophage polarization", JOURNAL OF CLINICAL INVESTIGATION, AMERICAN SOCIETY FOR CLINICAL INVESTIGATION, US, vol. 117, no. 1, 1 January 2007 (2007-01-01), pages 175-184, XP002508529, ISSN: 0021-9738, DOI: 10.1172/JCI29881
- Dissertation ET AL: "Adipositas: Inflammation im viszeralen Fettgewebe", , 1 January 2010 (2010-01-01), XP055262004, Retrieved from the Internet: URL:http://d-nb.info/1000962628/34/ [retrieved on 2016-04-01]
- Stulnig Tm: "Austrian Journal of Clinical Endocrinology and Metabolism Austrian Journal of Clinical Endocrinology and Metabolism Online-Datenbank mit Autoren-und Stichwortsuche Adipositas und die Entzündung des Fettgewebes", Journal of Clinical Endocrinology and Metabolism, 1 January 2009 (2009-01-01), pages 17-21, XP055262005, Retrieved from the Internet: URL:http://www.kup.at/kup/pdf/8176.pdf
- MINE S ET AL: "Increased expression levels of monocyte CCR2 and monocyte chemoattractant protein-1 in patients with diabetes mellitus", BIOCHEMICAL AND BIOPHYSICAL RESEARCH COMMUNICATIONS, ELSEVIER, AMSTERDAM, NL, vol. 344, no. 3, 9 June 2006 (2006-06-09), pages 780-785, XP024924961, ISSN: 0006-291X, DOI: 10.1016/J.BBRC.2006.03.197 [retrieved on 2006-06-09]

### SEQUENCE LISTING

<110> ITH Immune Therapy Holdings AB
<120> TREATING CONDITIONS ASSOCIATED WITH METABOLIC SYNDROME
<130> P117417WO00
<160> 17
<170> PatentIn version 3.5
<210> 1
   <211> 99
   <212> PRT
   <213> Artificial sequence
<220>
   <223> Synthesised peptide
<400> 1
   Met Lys Val Ser Ala Ala Leu Leu Cys Leu Leu Leu Ile Ala Ala Thr
   1               5                   10                  15
   Phe Ile Pro Gln Gly Leu Ala Gln Pro Asp Ala Ile Asn Ala Pro Val
               20                  25                  30
   Thr Cys Cys Tyr Asn Phe Thr Asn Arg Lys Ile Ser Val Gln Arg Leu
           35                  40                  45
   Ala Ser Tyr Arg Arg Ile Thr Ser Ser Lys Cys Pro Lys Glu Ala Val
       50                  55                  60
   Ile Phe Lys Thr Ile Val Ala Lys Glu Ile Cys Ala Asp Pro Lys Gln
   65                  70                  75                  80
   Lys Trp Val Gln Asp Ser Met Asp His Leu Asp Lys Gln Thr Gln Thr
                   85                  90                  95
   Pro Lys Thr
<210> 2
   <211> 76
   <212> PRT
   <213> Artificial sequence
<220>
   <223> Synthesised peptide
<220>
   <221> X
   <222> (64)..(64)
   <223> X = methionine or norleucine
<400> 2
   Gln Pro Asp Ala Ile Asn Ala Pro Val Thr Cys Cys Tyr Asn Phe Thr
   1               5                   10                  15
   Asn Arg Lys Ile Ser Val Gln Arg Leu Ala Ser Tyr Arg Arg Ile Thr
               20                  25                  30
   Ser Ser Lys Cys Pro Lys Glu Ala Val Ile Phe Lys Thr Ile Val Ala
           35                  40                  45
   Lys Glu Ile Cys Ala Asp Pro Lys Gln Lys Trp Val Gln Asp Ser Xaa
       50                  55                  60
   Asp His Leu Asp Lys Gln Thr Gln Thr Pro Lys Thr
   65                  70                  75
<210> 3
   <211> 68
   <212> PRT
   <213> Artificial sequence
<220>
   <223> Synthesised peptide
<400> 3
   Val Thr Cys Cys Tyr Asn Phe Thr Asn Arg Lys Ile Ser Val Gln Arg
   1               5                   10                  15
   Leu Ala Ser Tyr Arg Arg Ile Thr Ser Ser Lys Cys Pro Lys Glu Ala
               20                  25                  30
   Val Ile Phe Lys Thr Ile Val Ala Lys Glu Ile Cys Ala Asp Pro Lys
           35                  40                  45
   Gln Lys Trp Val Gln Asp Ser Met Asp His Leu Asp Lys Gln Thr Gln
       50                  55                  60
   Thr Pro Lys Thr
   65
<210> 4
   <211> 104
   <212> PRT
   <213> Artificial sequence
<220>
   <223> Synthesised peptide
<400> 4
   Met Lys Ile Ser Thr Leu Leu Cys Leu Leu Leu Ile Ala Thr Thr Ile
   1               5                   10                  15
   Ser Pro Gln Val Leu Ala Gly Pro Asp Ala Val Ser Thr Pro Val Thr
   20 25 30
   Cys Cys Tyr Asn Val Val Lys Gln Lys Ile His Val Arg Lys Leu Lys
   35 40 45
   Ser Tyr Arg Arg Ile Thr Ser Ser Gln Cys Pro Arg Glu Ala Val Ile
   50 55 60
   Phe Arg Thr Ile Leu Asp Lys Glu Ile Cys Ala Asp Pro Lys Glu Lys
   65 70 75 80
   Trp Val Lys Asn Ser Ile Asn His Leu Asp Lys Thr Ser Gln Thr Phe
   85 90 95
   Ile Leu Glu Pro Ser Cys Leu Gly
   100
<210> 5
   <211> 82
   <212> PRT
   <213> Artificial sequence
<220>
   <223> Synthesised peptide
<400> 5
   Gly Pro Asp Ala Val Ser Thr Pro Val Thr Cys Cys Tyr Asn Val Val
   1 5 10 15
   Lys Gln Lys Ile His Val Arg Lys Leu Lys Ser Tyr Arg Arg Ile Thr
   20 25 30
   Ser Ser Gln Cys Pro Arg Glu Ala Val Ile Phe Arg Thr Ile Leu Asp
   35 40 45
   Lys Glu Ile Cys Ala Asp Pro Lys Glu Lys Trp Val Lys Asn Ser Ile
   50 55 60
   Asn His Leu Asp Lys Thr Ser Gln Thr Phe Ile Leu Glu Pro Ser Cys
   65 70 75 80
   Leu Gly
<210> 6
   <211> 76
   <212> PRT
   <213> Artificial sequence
<220>
   <223> Synthesised peptide
<400> 6
   Gly Pro Asp Ala Val Ser Thr Pro Val Thr Cys Cys Tyr Asn Val Val
   1               5                   10                  15
   Lys Gln Lys Ile His Val Arg Lys Leu Lys Ser Tyr Arg Arg Ile Thr
               20                  25                  30
   Ser Ser Gln Cys Pro Arg Glu Ala Val Ile Phe Arg Thr Ile Leu Asp
           35                  40                  45
   Lys Glu Ile Cys Ala Asp Pro Lys Glu Lys Trp Val Lys Asn Ser Ile
       50                  55                  60
   Asn His Leu Asp Lys Thr Ser Gln Thr Phe Ile Leu
   65                  70                  75
<210> 7
   <211> 76
   <212> PRT
   <213> Artificial sequence
<220>
   <223> Synthesised peptide
<400> 7
   Gly Pro Asp Ala Val Ser Thr Pro Val Thr Cys Cys Tyr Asn Val Val
   1               5                   10                  15
   Lys Gln Lys Ile His Val Arg Lys Leu Lys Ser Tyr Arg Arg Ile Thr
               20                  25                  30
   Ser Ser Gln Cys Pro Arg Glu Ala Val Ile Phe Arg Thr Ile Leu Asp
           35                  40                  45
   Lys Glu Ile Cys Ala Asp Pro Lys Glu Lys Trp Val Lys Asn Ser Ile
       50                  55                  60
   Asn His Leu Asp Lys Thr Ser Gln Thr Phe Lys Leu
   65                  70                  75
<210> 8
   <211> 68
   <212> PRT
   <213> Artificial sequence
<220>
   <223> Synthesised peptide
<400> 8
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Glu Lys Ser
   65
<210> 9
   <211> 76
   <212> PRT
   <213> Artificial sequence
<220>
   <223> Synthesised peptide
<220>
   <221> X
   <222> (1)..(1)
   <223> X = pyroGlu or Gln
<400> 9
   Xaa Pro Asp Ala Ile Asn Ala Pro Val Thr Cys Cys Tyr Asn Phe Thr
   1               5                   10                  15
   Asn Arg Lys Ile Ser Val Gln Arg Leu Ala Ser Tyr Arg Arg Ile Thr
               20                  25                  30
   Ser Ser Lys Cys Pro Lys Glu Ala Val Ile Phe Lys Thr Ile Val Ala
           35                  40                  45
   Lys Glu Ile Cys Ala Asp Pro Lys Gln Lys Trp Val Gln Asp Ser Met
       50                  55                  60
   Asp His Leu Asp Lys Gln Thr Gln Thr Pro Lys Thr
   65                  70                  75
<210> 10
   <211> 76
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (75)..(75)
   <223> X = K(ivDde)
<400> 10
   Xaa Pro Asp Ala Ile Asn Ala Pro Val Thr Cys Cys Tyr Asn Phe Thr
   1               5                   10                  15
   Asn Arg Lys Ile Ser Val Gln Arg Leu Ala Ser Tyr Arg Arg Ile Thr
               20                  25                  30
   Ser Ser Lys Cys Pro Lys Glu Ala Val Ile Phe Lys Thr Ile Val Ala
           35                  40                  45
   Lys Glu Ile Cys Ala Asp Pro Lys Gln Lys Trp Val Gln Asp Ser Met
       50                  55                  60
   Asp His Leu Asp Lys Gln Thr Gln Thr Pro Xaa Thr
   65                  70                  75
<210> 11
   <211> 76
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (75)..(75)
   <223> X is an amino acid residue that can be biotinylated, such as lysine, ornithine or diaminopropionic acid and optionally is biotinylated, optionally via a spacer molecule such as PEG
<400> 11
   Xaa Pro Asp Ala Ile Asn Ala Pro Val Thr Cys Cys Tyr Asn Phe Thr
   1 5 10 15
   Asn Arg Lys Ile Ser Val Gln Arg Leu Ala Ser Tyr Arg Arg Ile Thr
   20 25 30
   Ser Ser Lys Cys Pro Lys Glu Ala Val Ile Phe Lys Thr Ile Val Ala
   35 40 45
   Lys Glu Ile Cys Ala Asp Pro Lys Gln Lys Trp Val Gln Asp Ser Met
   50 55 60
   Asp His Leu Asp Lys Gln Thr Gln Thr Pro Xaa Thr
   65 70 75
<210> 12
   <211> 68
   <212> PRT
   <213> Artificial sequence
<220>
   <223> Synthesised peptide
<400> 12
   Ser Pro Tyr Ser Ser Asp Thr Thr Pro Cys Cys Phe Ala Tyr Ile Ala
   1 5 10 15
   Arg Pro Leu Pro Arg Ala His Ile Lys Glu Tyr Phe Tyr Thr Ser Gly
   20 25 30
   Lys Cys Ser Asn Pro Ala Val Val Phe Val Thr Arg Lys Asn Arg Gln
   35 40 45
   Val Cys Ala Asn Pro Glu Lys Lys Trp Val Arg Glu Tyr Ile Asn Ser
   50 55 60
   Leu Glu Lys Ser
   65
<210> 13
   <211> 68
   <212> PRT
   <213> Artificial sequence
<220>
   <223> Synthesised peptide
<220>
   <221> X
   <222> (67)..(67)
   <223> X is K(ivDde)
<400> 13
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Glu Xaa Ser
   65
<210> 14
   <211> 68
   <212> PRT
   <213> Artificial sequence
<220>
   <223> Synthesised peptide
<220>
   <221> X
   <222> (67)..(67)
   <223> X is an amino acid residue that can be biotinylated, such as lysine, ornithine or diaminopropionic acid and optionally is biotinylated, optionally via a spacer molecule such as PEG (e.g. K(Biotin))
<400> 14
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Glu Xaa Ser
   65
<210> 15
   <211> 76
   <212> PRT
   <213> Artificial sequence
<220>
   <223> Synthesised peptide
<220>
   <221> X
   <222> (1)..(1)
   <223> X = pyroGlu or Gln
<400> 15
   Xaa Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
           35                  40                  45
   Lys Glu Val Cys Ala Asp Pro Lys Glu Arg Trp Val Arg Asp Ser Met
       50                  55                  60
   Lys His Leu Asp Gln Ile Phe Gln Asn Leu Lys Pro
   65                  70                  75
<210> 16
   <211> 76
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (75)..(75)
   <223> X = K(ivDde)
<400> 16
   Xaa Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
           35                  40                  45
   Lys Glu Val Cys Ala Asp Pro Lys Glu Arg Trp Val Arg Asp Ser Met
       50                  55                  60
   Lys His Leu Asp Gln Ile Phe Gln Asn Leu Xaa Pro
   65                  70                  75
<210> 17
   <211> 76
   <212> PRT
   <213> Artificial sequence
<220>
   <223> Synthesised peptide
<220>
   <221> X
   <222> (1)..(1)
   <223> X = pyroGlu or Gln
<220>
   <221> X
   <222> (75)..(75)
   <223> X is an amino acid residue that can be biotinylated, such as lysine, ornithine or diaminopropionic acid and optionally is biotinylated, optionally via a spacer molecule such as PEG
<400> 17
   Xaa Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
           35                  40                  45
   Lys Glu Val Cys Ala Asp Pro Lys Glu Arg Trp Val Arg Asp Ser Met
       50                  55                  60
   Lys His Leu Asp Gln Ile Phe Gln Asn Leu Xaa Pro
   65                  70                  75

## Description

### FIELD OF THE INVENTION

The various embodiments of the present invention relates to products for use in treating diabetes.

### BACKGROUND OF THE INVENTION

The International Diabetes Foundation (IDF) define metabolic syndrome as follows:
For a person to be defined as having the metabolic syndrome they must have:
Central obesity (defined as waist circumference ≥ 94cm for Europid men and ≥ 80cm for Europid women, with ethnicity specific values for other groups)
plus any two of the following four factors:
   - raised triglyceride (TG) level: ≥ 150 mg/dL (1.7 mmol/L), or specific treatment for this lipid abnormality
   - reduced high-density lipoprotein (HDL) cholesterol: < 40 mg/dL (1.03 mmol/L*) in males and < 50 mg/dL (1.29 mmol/L*) in females, or specific treatment for this lipid abnormality
   - raised blood pressure (BP): systolic BP ≥ 130 or diastolic BP ≥ 85 mm Hg, or treatment of previously diagnosed hypertension
   - raised fasting plasma glucose (FPG) ≥ 100 mg/dL (5.6 mmol/L), or previously diagnosed type 2 diabetes
If above 5.6 mmol/L or 100 mg/dL, oral glucose tolerance test (OGTT) is strongly recommended but is not necessary to define presence of the syndrome.

While the pathogenesis of the metabolic syndrome and each of its components is complex and not well understood, central obesity and insulin resistance are acknowledged as important causative factors.

Adiposis dolorosa, or Dercum's disease, is a rare progressive condition characterized by multiple, painful, subcutaneous lipomas that usually occur in obese, postmenopausal women (Dercum FX. Three cases of a hitherto unclassified affection resembling in its grosser aspects obesity, but associated with special symptoms: adiposis dolorosa. Am J Med Sci 1892; 104:521-35).

Apheresis is a treatment used for depletion of blood components, such as antibodies, low-density lipoproteins (LDL) and blood cells. Leukapheresis is the apheresis treatment used for removal of white blood cells, leukocytes. The patient is connected to an extracorporeal blood circulating system; the blood is drawn from a vein in one arm, passed through a column device and returned into the other arm of the patient. WO2010/029317 describes apheresis columns useful for treating inflammatory conditions including a chemokine immobilised on a solid support. Moreover, EP 2 067 495 relates to the provision of a cell adsorption column which has an adsorbent with a surface area where there is less concern about denaturation and differentiation induction of cells caused by a contact of the adsorbent with the cells, and whose adsorbent-filled volume is small, and which has a compact property for improving operability and reducing a burden on a patient. Thus EP 2 067 495 provides a cell adsorption column filled with an adsorbent containing a fiber whose fiber diameter is 0.5 µm or more and 10 µm or less and having a surface area of 0.5 m² or more and less than 10m², in which the adsorbent-filled volume is 100 mL or less.

### SUMMARY OF THE INVENTION

Chemokines are a class of cytokine molecules involved in cell recruitment and activation in inflammation. Chemokines cause chemotaxis and activation of various subpopulations of cells in the immune system. The activity of chemokines is mediated primarily through tight binding to their receptors on the surface of leukocytes. In certain embodiments the present invention is based on the realisation that the interaction between chemokines and cells expressing their receptors may be exploited for the treatment of specific inflammatory conditions associated with metabolic syndrome. The inventors have determined that targeting increased recruitment of specific chemokine receptor-expressing cells to the site of inflammation presents a new therapeutic approach to treat such conditions.

It is shown herein that pro-inflammatory CCR2 expressing monocytes (such as macrophages) can be depleted from diabetes patients using a suitable chemokine such as MCP-1 (CCL2), in particular biotinylated MCP-1. This provides inflamed tissue, such as adipose and liver tissue, with the opportunity to heal and helps to prevent the development of insulin resistance.

Thus, in certain embodiments the invention serves to reduce the recruitment of inflammatory leukocytes, which express characteristic chemokine receptors, and possibly express characteristic chemokine receptors at increased levels, to sites of inflammation linked to disorders such as diabetes. This is achieved using specific binding reagents to capture specific chemokine receptor-expressing inflammatory leukocytes from the patient.

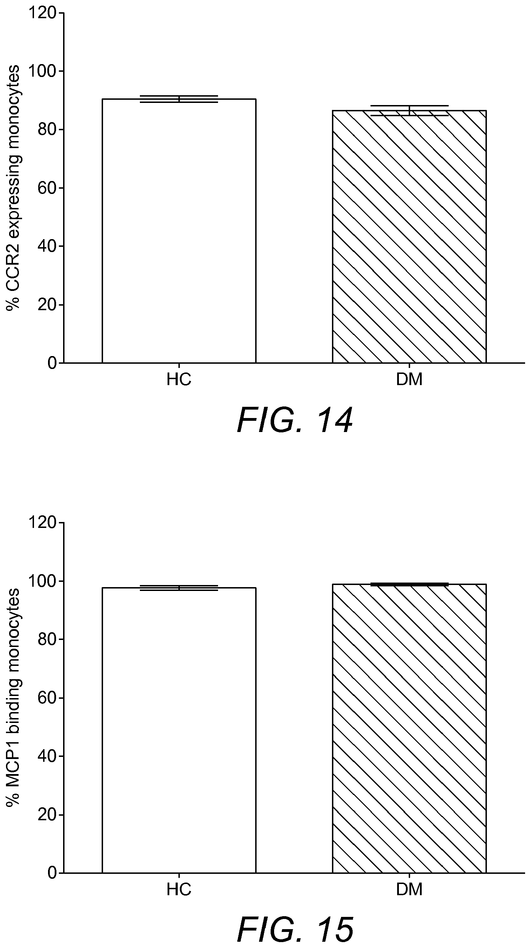

As shown herein, suitable binding reagents can be immobilized onto a solid support, either directly or indirectly, to generate an apheresis column suitable for capturing relevant chemokine receptor-expressing cells. Where increased levels of chemokine receptor expression are observed, such cells may be preferably removed from the peripheral blood using the columns of the various embodiments of the invention. Thus, the methods of the various embodiments of the invention may preferably target CCR2^{hi} cells as defined herein for removal from the peripheral blood. "High" expression may be determined according to standard flow cytometry techniques. The level may be measured relative to levels of expression of the chemokine receptor in cells taken from a healthy subject. The attached Figure 15 provides an example of a gating strategy.

In other embodiments the invention further provides a binding reagent capable of specifically binding to the chemokine receptor CCR2 for use in the treatment of a diabetes condition, wherein the one or more binding reagents is immobilized, directly or indirectly, on a solid support contained within an apheresis column, to which is applied peripheral blood from a patient thus removing chemokine receptor CCR2 expressing cells from the peripheral blood of the patient, and wherein the binding reagent is MCP-1.

Consequently, present invention relates to a binding reagent capable of specifically binding to the chemokine receptor CCR2 for use in the treatment of a diabetes condition, wherein the binding reagent is immobilized on a solid support contained within an apheresis column, to which is applied peripheral blood from a patient thus removing CCR2 expressing cells from the peripheral blood of the patient, and wherein the binding reagent is MCP-1.

In one aspect, present invention relates to a modified MCP-1 chemokine comprising the amino acid sequence set forth as SEQ ID NO: 1, SEQ ID NO: 2 or SEQ ID NO: 3 in which one or more of the following modifications have been made:
a) the glutamine residue at position 1 of SEQ ID NO: 2 has been replaced with pyroglutamine,
b) the C terminus is produced as an amide derivative,
c) the lysine residue at position 98 of SEQ ID NO: 1 or position 75 of SEQ ID NO:2 or position 67 of SEQ ID NO: 3, which may be replaced by a suitable alternative amino acid such as diaminopropionic acid or ornithine, is biotinylated, optionally via a spacer group, in order to permit immobilization of the chemokine on a solid support,
d) the methionine residue at position 87 of SEQ ID NO: 1 or position 64 of SEQ ID NO: 2 or position 56 of SEQ ID NO: 3 has been replaced with norleucine.

In a further aspect, present invention relates to a modified CCL2 chemokine comprising the amino acid sequence of SEQ ID NO: 9 or SEQ ID NO: 11.

Monocytes are produced by the bone marrow from haematopoietic stem cell precursors called monoblasts. Monocytes may differentiate into macrophages or dendritic cells. Monocytes and their macrophage and dendritic cell progeny serve a number of functions in the immune system including phagocytosis, antigen presentation and cytokine production. Monocytes may be characterized with reference to expression of the cell surface marker CD14, optionally together with CD16. Classical monocytes may be characterized by high level expression of the CD14 cell surface receptor (CD14++ CD16- monocyte). Non-classical monocytes may be characterized by low level expression of CD14 and with additional co-expression of the CD16 receptor (CD14+CD16++ monocyte). Intermediate monocytes may be characterized by high level expression of CD14 and low level expression of CD16 (CD14++CD16+ monocytes). Macrophages are derived from monocytes and are responsible for protecting tissues from foreign substances. They are cells that possess a large smooth nucleus, a large area of cytoplasm and internal vesicles for processing foreign material. The term "macrophage" may refer to a monocyte-derived cell expressing one or more of the following cell surface markers CD14, CD11b, Lysozyme M, MAC-1/MAC-3 and CD68. The term macrophage includes both activated and un-activated macrophages. Activated macrophages may be characterized by expression of one or more of CD69, ENG, FCER2 and IL2RA, HLA-DR, CD86. Un-activated macrophages have not yet received activating signals through for example TLR receptors and therefore they express less activation markers on the cell surface which correlates with lesser maturation. M1 macrophages may be characterized by expression of one or more of CD16⁺CD32⁺CD64⁺ and secrete mainly IL-23 and IL-1, TNF, IL-6 and high levels of IL-12 and in addition effector molecules such as iNOS and ROI. M1 macrophages have cytotoxic features as opposed to M2 macrophages. M2 macrophages may be characterized by expression of one or more of SRA/B⁺CD163⁺MR⁺CD14⁺ and express TGFβ, IL-10 and IL-1Ra. Tumour associated macrophages (TAMs) share many characteristics with the M2 macrophages and are considered as M2 polarised macrophages. The M1/M2 paradigm can also be found in monocyte subsets where CD14⁺C016⁻CXC3R1^{low} monocytes are considered the "inflammatory" subset and the CD14^{low}CD16⁺CXC3R1^{high} are "resident" monocytes.

The three major types of lymphocyte are T cells, B cells and natural killer (NK) cells. The term "T-lymphocyte" includes CD4⁺ T cells such as T helper cells (Th1 cells and Th2 cells), and CD8⁺ T cells such as cytotoxic T cells. Th1 cells may be characterized by expression of CCR5 and/or by production of IFN-γ. Th2 cells may be characterized by expression of CCR3 and/or by production of IL-4.

CCR2, CCR1, CCR3, CCR4 or CCR5 expressed on these aforementioned cells binds to chemokines such as monocyte chemoattractant protein-1 (MCP-1, CCL2) or CCL5. MCP-1 is a major chemoattractant for monocytes and memory T cells by means of their binding to its specific cell-surface receptor, CC-chemokine receptor-2. CCL5 is the gene symbol approved by the HUGO Gene Nomenclature Committee for chemokine (C-C motif) ligand 5, also known as RANTES. The HGNC ID for this gene is 10632. The gene is located at chromosome position 17q11.2-q12. The previous symbol and name for the gene is D17S136E, SCYA5, "small inducible cytokine A5 (RANTES)". Synonyms for this gene include "beta-chemokine RANTES", MGC17164, RANTES, "regulated upon activation, normally T-expressed, and presumably secreted", "SIS-delta", SISd, "small inducible cytokine subfamily A (Cys-Cys), member 5", "T-cell specific protein p288", "T-cell specific RANTES protein", and TCP228. The Genbank reference sequence for CCL5 is AF043341.1. RANTES binds to CCR1, CCR3 or CCR5.

CCR1 is the gene symbol approved by the HUGO Gene Nomenclature Committee for chemokine (C-C motif) receptor 1. The HGNC ID for this gene is 1602. The gene is located at chromosome position 3p21. The previous symbol and name CMKBR1, SCYAR1. Synonyms for this gene include CD191, CKR-1, MIP1α R. The Entrez Gene reference sequence for CCR1 is 1230 as available on 13 June 2011.

CCR2 is the gene symbol approved by the HUGO Gene Nomenclature Committee for chemokine (C-C motif) receptor 2. The HGNC ID for this gene is 1603. The gene is located at chromosome position 3p21. The previous symbol and name for the gene is CMKBR2. Synonyms for this gene include CC-CKR-2, CD192, CKR2, FLJ78302, MCP-1-R. The NCBI Reference Sequence is NM_001123041.2 as available on 13 June 2011.

CCR3 is the gene symbol approved by the HUGO Gene Nomenclature Committee for chemokine (C-C motif) receptor 3. The HGNC ID for this gene is 1604. The gene is located at chromosome position 3p21.3. The previous symbol and name for the gene is CMKBR3. Synonyms for this gene include CC-CKR-3, CD193 and CKR3. The Genbank reference sequence for CCR3 is AF247361.1 as available on 13 June 2011.

CCR5 is the gene symbol approved by the HUGO Gene Nomenclature Committee for chemokine (C-C motif) receptor 5. The HGNC ID for this gene is 1605. The gene is located at chromosome position 3p21. The previous symbol and name for the gene is CMKBR5. Synonyms for this gene include CC-CKR-5, CD195 CKR-5, IDDM22 and CKR5. The Entrez Gene reference sequence for CCR5 is 1234 as available on 13 June 2011.

CCR4 is the gene symbol approved by the HUGO Gene Nomenclature Committee for chemokine (C-C motif) receptor 4. The HGNC ID for this gene is 1605. The gene is located at chromosome position 3p24-p21.3. Synonyms for this gene include CC-CKR-4, CD194, ChemR13, CKR4, CMKBR4, k5-5. The Genbank reference sequence for CCR4 is X85740.1 as available on 13 June 2011.

It has been shown that CCR2 expression on monocytes is elevated in diabetic patients. Furthermore, transgenic mice with an adipose-tissue-specific expression of MCP-1 have macrophage infiltration into adipose tissue, increased hepatic triacylglycerol content and insulin resistance. MCP-1-knockout mice fed a high-fat diet have a drastically reduced macrophage accumulation into adipose tissue and hepatic steatosis when compared with high-fat fed wild-type mice. Inhibition of MCP-1 function by the acute expression of a dominant negative mutant of MCP-1 ameliorated insulin resistance in db/db mice and in high-fat-fed wild-type mice. Moreover, an increased level of serum MCP-1 correlates with conditions associated with metabolic syndrome, including obesity, insulin resistance, Type 2 diabetes, hypertension and increased serum triacylglyerol concentrations. It is shown herein that MCP-1 can be used to reduce CCR2-expressing monocyte levels in diabetic subjects. This provides inflamed tissue, such as adipose and liver tissue, with the opportunity to heal and helps to prevent the development of insulin resistance.

Present invention relies upon a binding reagent which is capable of specifically binding to a chemokine receptor. This specific binding reaction permits cells expressing the chemokine receptor to be removed from the peripheral blood of the patient when the blood is passed over the solid support upon or within which the binding reagent is immobilized. Specific chemokine receptors of interest include CCR2 By "specific binding" is meant that the binding reagent displays sufficient specificity of binding and appropriate binding affinity/kinetics to permit removal of cells expressing CCR2 from the peripheral blood. The binding reagent capable of specifically binding to CCR2 may be either an agonist or an antagonist of CCR2. As the disease condition relies upon up-regulation of expression of or signaling via CCR2 in certain embodiments the binding reagent capable of specifically binding to CCR2 is an antagonist of CCR2. Chemokines are typically, although not necessarily exclusively (particularly in the case of truncated or modified forms) agonists of their cognate receptor and serve to activate the cells expressing the relevant receptor, as would be appreciated by one skilled in the art. Specific examples of binding reagents are chemokines.

In the context of the various embodiments of the present invention the term "chemokine" also comprises biotinylated or otherwise labeled chemokines. The term "chemokine" also comprises modified and truncated versions of the chemokine and chemokine fragments with the proviso that the modified or truncated form retains its ability to bind to its cognate receptor (and thus remains functional in the context of the various embodiments of the invention). The chemokine does not necessarily need to retain biological activity as it is specific binding affinity for CCR2 that is required. In certain embodiments, the chemokine lacks biological activity, for example in terms of activation of the CCR2 receptor. Modifications may be made to improve protein synthesis, for example uniformity of product and yield. As known to those skilled in the art, exemplary modifications may comprise amino acid additions, substitutions, deletions or other modifications to one or more amino acids in the chemokine. Modifications may comprise substitution of the wild type amino acid with non-natural amino acids such as norleucine (NLeu) and derivatized amino acids such as pyroglutamic acid (pyroGlu). Such modifications may be made to minimize side-product formation during storage and use of the columns of the various embodiments of the invention. Modifications may be made to improve labelling, for example inclusion of a polyethylene glycol (PEG) spacer to facilitate biotinylation. The biotinylation and/or conjugation with fluorochromes or other labelling groups of the chemokine is performed in a manner which does not substantially affect the receptor binding capacity. Site specific biotinylation or other labelling is preferred as non-selective labelling of chemokines may compromise receptor binding activity. Biotinylation or other labelling is generally preferred at or towards the C-terminus of the protein as the inventors have found that modifications in this area are generally well tolerated (in terms of a minimal effect on receptor binding capability). Biotinylation may be carried out site-specifically at any suitable amino acid. Examples of suitable amino acids include lysine, diaminopropionic acid and ornithine. Generally, reference may be made to Natarajan S et al, Int. J. Pept. Protein Res., 1992, 40, 567-74; Baumeister B, Int. J. Peptide Res. And Therapeutics, 2005, 11, 139-141; Bioconjugate techniques 2nd edition, Greg T. Hermanson.

Truncations may involve deletion of either N or C terminal amino acids as appropriate, or both. Typically, the truncated version will retain the residues required for the chemokine to fold correctly, for example to retain a chemokine fold structure, consistent with the requirement that a truncated version must retain the ability to bind to the relevant receptor (expressed by (on the surface of) a leukocyte). Chemokine molecules typically include disulphide bonds between the 1^{st} and 3^{rd} and 2^{nd} and 4^{th} cysteine residues respectively, as would be understood by one skilled in the art. Where sequences are presented herein, it is assumed that these disulphide bonds will form in the folded protein (unless otherwise stated).

Specific chemokines useful in the various embodiments of the present invention for binding to CCR2 include MCP-1. MCP-1 binds solely to the chemokine receptor CCR2 and so this chemokine is preferred. More specifically, MCP-1 is useful for removing CCR2 expressing cells from the blood of a patient.

The modified and truncated chemokines described in greater detail herein (with reference to the relevant amino acid sequences, as set forth in the SEQ ID NOs and accompanying experimental examples). Such modified forms may instruct the skilled person regarding additional modified forms of the same and other chemokines which may be suitable for use in the invention. Chemokines show variable sequence homology varying from less than 20% to over 90% but all share very similar tertiary structures consisting of a disordered N-terminus, followed by a long loop (the N-loop) that ends in a 3₁₀ helix, a 3-stranded β-sheet and a C-terminal helix. The overall topology is stabilsed by disulphide bonds. This common tertiary structure is a common feature of the chemokine protein family (Fernandez EJ annd Lolis E., Annu. Rev. Pharmacol. Toxicol., 202, 42, 469-99; Allen SJ et al, Annu. Rev. Immunol., 2007, 25, 787-820).

Truncations within this N-terminal region can maintain binding to the receptor but can lead to a change or loss of function (for examples Zhang YJ et al, J. Biol. Chem., 1994, 269, 15918, ; Gong J-H and Clark-Lewis I., J. Exp. Med., 1995, 181, 631-640; Fernandez EJ annd Lolis E., Annu. Rev. Pharmacol. Toxicol., 202, 42, 469-99; Allen SJ et al, Annu. Rev. Immunol., 2007, 25, 787-820). Truncations at the C-terminus of the chemokine can also be made and maintain receptor binding activity (Treating Inflammatory Disorders, Ola Winqvist and Graham Cotton, WO2010/029317).

Chemokines can be modified by a variety of chemical techniques to produce derivatives having essentially the same activity or function-such as binding to a cognate receptor-as the unmodified peptides, and optionally having other desirable properties. For example, carboxylic acid groups of the protein, whether carboxyl-terminal or side chain, may be provided in the form of a salt of a pharmaceutically-acceptable cation or esterified to form a C1-C16 ester, or converted to an amide of formula NR1R2 wherein R1 and R2 are each independently H or C1-C16 alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCI, HBr, acetic, benzoic, toluene sulfonic, maleic, tartaric and other organic salts, or may be modified to C1-C16 alkyl or dialkyl amino or further converted to an amide.

Hydroxyl groups of the peptide side chains can be converted to C1-C16 alkoxy or to a C1-C16 ester using well-recognized techniques. Phenyl and phenolic rings of the peptide side chains can be substituted with one or more halogen atoms, such as F, Cl, Br or I, or with C1-C16 alkyl, C1-C16 alkoxy, carboxylic acids and esters thereof, or amides of such carboxylic acids. Methylene groups of the peptide side chains can be extended to homologous C2-C4 alkylenes. Thiols can be protected with any one of a number of well-recognized protecting groups, such as acetamide groups. Those skilled in the art will also recognize methods for introducing cyclic structures into the peptides of this disclosure to select and provide conformational constraints to the structure that result in enhanced stability. For example, a C- or N-terminal cysteine can be added to the peptide, so that when oxidized the peptide will contain a disulfide bond, generating a cyclic peptide. Other peptide cyclizing methods include the formation of thioethers and carboxyl- and amino-terminal amides and esters.

Amino acids in a peptide, polypeptide, or protein generally are chemically bound together via amide linkages (CONH). Additionally, amino acids may be bound together by other chemical bonds. For example, linkages for amino acids or amino acid analogs can include CH2NH-, - CH2S-, -CH2-CH2 -, -CH=CH-- (cis and trans), -COCH2 --, -CH(OH)CH2-, and -CHH2SO-(These and others can be found in Spatola, in Chemistry and Biochemistry of Amino Acids, Peptides, and Proteins, B. Weinstein, eds., Marcel Dekker, New York, p. 267 (1983); Spatola, A. F., Vega Data (March 1983), Vol. 1, Issue 3, Peptide Backbone Modifications (general review); Morley, Trends Pharm Sci pp. 463-468, 1980; Hudson, et al., Int J Pept Prot Res 14:177-185, 1979; Spatola et al. Life Sci 38:1243-1249, 1986; Harm J. Chem. Soc Perkin Trans. 1307-314, 1982; Almquist et al. J. Med. Chem. 23:1392-1398, 1980; Jennings-White et al. Tetrahedron Lett 23:2533, 1982; Holladay et al. Tetrahedron. Lett 24:4401-4404, 1983; and Hruby Life Sci 31:189-199, 1982.

CCL2 is the gene symbol approved by the HUGO Gene Nomenclature Committee for chemokine (C-C motif) ligand 2, also known as MCP-1. The HGNC ID for this gene is 10618. The gene is located at chromosome position 17q11.2-q21.1. The previous symbol and name for the gene is SCYA2 "small inducible cytokine A2 (monocyte chemotatic protein 1, homologus to mouse Sig-je)". Synonyms for this gene include GDCF-2, HC11, MCP1, MGC9434, SMC-CF, "monocyte chemoattractant protein-1", "monocyte chemotactic and activating factor", "monocyte chemotactic protein 1, homologous to mouse Sig-je", "monocyte secretory protein JE", "small inducible cytokine subfamily A (Cys-Cys), member 2". The Genbank reference sequence for CCL2 is BC009716.1 as available on 13 June 2011.

CCL8 is the gene symbol approved by the HUGO Gene Nomenclature Committee for chemokine (C-C motif) ligand 8, also known as MCP-2. The HGNC ID for this gene is 10635. The gene is located at chromosome position 17q11.2. The previous symbol and name for the gene is SCYA8, "small inducible cytokine subfamily A (Cys-Cys), member 8 (monocyte chemotactic protein 2)". Another synonym for this gene is HC14. The Genbank reference sequence for CCL8 is X99886.1 as available on 13 June 2011.

CCL7 is the gene symbol approved by the HUGO Gene Nomenclature Committee for chemokine (C-C motif) ligand 7, also known as MCP-3. The HGNC ID for this gene is 10634. The gene is located at chromosome position 17q11.2-q12. The previous symbol and name for the gene is SCYA6, SCYA7, "small inducible cytokine A7 (monocyte chemotactic protein 3)". Synonyms for this gene include FIC, MARC, MCP-3, MCP3, NC28, "monocyte chemoattractant protein 3", "monocyte chemotactic protein 3". The Genbank reference sequence for CCL7 is AF043338 as available on 13 June 2011.

CCL13 is the gene symbol approved by the HUGO Gene Nomenclature Committee for chemokine (C-C motif) ligand 13, also known as MCP-4. The HGNC ID for this gene is 10611. The gene is located at chromosome position 17q11.2. The previous symbol and name for the gene is SCYA13, "small inducible cytokine subfamily A (Cys-Cys), member 13". Synonyms for this gene include CKb10, MCP-4, MGC17134, NCC-1, SCYL1. The Genbank reference sequence for CCL13 is AJ001634 as available on 13 June 2011.

MCP-5 is a mouse chemokine in the CC chemokine family. It is also known as Chemokine (C-C motif) ligand 12 (CCL12) and, due to its similarity with the human chemokine MCP-1, sometimes it is called MCP-1-related chemokine. The gene for MCP-5 is found in a cluster of CC chemokines on mouse chromosome 11. The NCBI reference sequence for CCL12 is NC_000077.5. The previous symbol for MCP-5 is SCYA12. The NCBI reference sequence for CCL12 is NC_000077.5 as available on 13 June 2011.

CCL5 is the gene symbol approved by the HUGO Gene Nomenclature Committee for chemokine (C-C motif) ligand 5, also known as RANTES. The HGNC ID for this gene is 10632. The gene is located at chromosome position 17q11.2-q12. The previous symbol and name for the gene is D17S136E, SCYA5, "small inducible cytokine A5 (RANTES)". Synonyms for this gene include "beta-chemokine RANTES", MGC17164, RANTES, "regulated upon activation, normally T-expressed, and presumably secreted", "SIS-delta", SISd, "small inducible cytokine subfamily A (Cys-Cys), member 5", "T-cell specific protein p288", "T-cell specific RANTES protein", TCP228. The Genbank reference sequence for CCL5 is AF043341.1 as available on 13 June 2011.

CCL17 is the gene symbol approved by the HUGO Gene Nomenclature Committee for chemokine (C-C motif) ligand 17. The HGNC ID for this gene is 10615. The gene is located at chromosome position 16q13. The previous symbol and name for the gene is SCYA17, "small inducible cytokine subfamily A (Cys-Cys), member 17". Synonyms for this gene include ABCD-2, TARC. The Genbank reference sequence for CCL17 is D43767.1 as available on 13 June 2011.

CCL21 is the gene symbol approved by the HUGO Gene Nomenclature Committee for chemokine (C-C motif) ligand 21. The HGNC ID for this gene is 10620. The gene is located at chromosome position 9p13. The previous symbol and name for the gene is SCYA21, "small inducible cytokine subfamily A (Cys-Cys), member 21". Synonyms for this gene include 6Ckine, "beta chemokine exodus-2", CKb9, ECL, "Efficient Chemoattractant for Lymphocytes", exodus-2, "secondary lymphoid tissue chemokine", SLC, TCA4. The Genbank reference sequence for CCL21 is AB002409.1 as available on 13 June 2011.

Examples of suitable modified chemokines of the various embodiments of the invention containing modifications and/or truncations and specifically adapted for use according to the invention are described in detail herein. MCP-1 has been produced with residue 75, which may be a lysine, as the site of biotinylation on the chemokine (numbering based upon the mature protein having the amino acid sequence of SEQ ID NO: 2). Biotinylation permits immobilization of MCP-1 on a solid support (via a biotin-avidin interaction). The basic amino acid sequence of MCP-1, including a 23 amino acid leader sequence is set forth as SEQ ID NO: 1. The amino acid sequence of the mature protein is set forth as SEQ ID NO: 2. The inventors have determined that chemokines may display improved binding properties where the chemokine is biotinylated via a spacer group. The spacer may prevent the biotin group from impacting on the binding affinity of the chemokine. Any suitable spacer group may be employed. Further modifications may provide the molecule with advantageous properties. The invention also relates to derivatives of truncated MCP-1 chemokines. The amino acid sequence of the truncated version is set forth as SED ID NO: 3.

Accordingly, in certain embodiments the invention also provides a modified MCP-1 chemokine comprising, consisting essentially of or consisting of the amino acid sequence set forth as SEQ ID NO: 1, SEQ ID NO: 2 or SEQ ID NO: 3 in which one or more of the following modifications have been made:
a) the glutamine residue 1 of SEQ ID NO: 2 has been replaced with pyroglutamine
b) the C terminus is produced as an amide derivative (this may be achieved by synthesis on an amide linker)
c) the (C terminal region) residue at position 98 of SEQ ID NO: 1 or position 75 of SEQ ID NO:2 or position 67 of SEQ ID NO: 3, which may be a lysine or ornithine residue, is biotinylated, optionally via a spacer group, in order to permit immobilization of the chemokine on a solid support; and/or
d) the methionine residue at position 87 of SEQ ID NO: 1 or position 64 of SEQ ID NO: 2 or position 56 of SEQ ID NO: 3 has been replaced with norleucine.

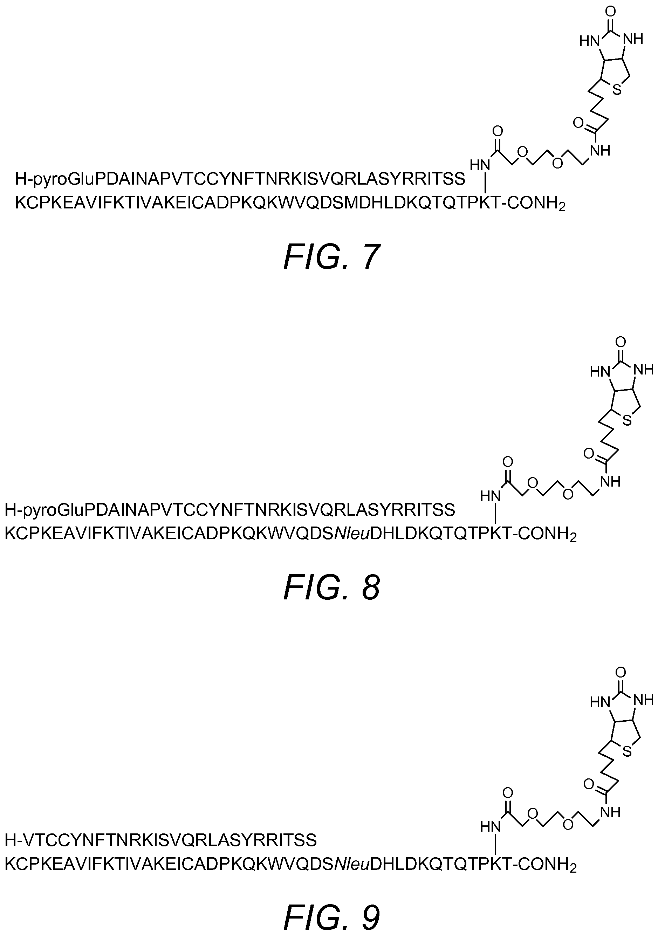

The (C terminal region) amino acid, which may be a lysine residue or a functional equivalent, at position 98 of SEQ ID NO: 1 or position 75 of SEQ ID NO:2 or position 67 of SEQ ID NO: 3 may be biotinylated via a suitable spacer group, such as a polyethylene glycol (PEG) spacer group, in order to permit immobilization of the chemokine on a solid support. In specific embodiments, the PEG spacer is 3,6-dioxo aminooctanoic acid. The sequence and biotinylation of the modified MCP-1 chemokines of the invention are shown in figures 7 to 9 respectively. The modified MCP-1 chemokines may be agonists or antagonists of CCR2 activity. They can be tested for activity in a suitable assay, such as cell-based assays. In particular, agonist and antagonist properties may be determined in functional cell-based assay on human CCR2 receptor.

A further example of a chemokine of the various embodiments of the invention containing modifications and specifically adapted for use in the invention is described in detail herein (see Example below). The modified CCL2 (MCP-1) corresponds to residues 1 to 76 of the full length mature protein (and lacks the N-terminal signal peptide of 23 amino acids, which is cleaved off) and thus retains the chemokine fold. The Gln at the N-terminus of the protein (Gln1) is substituted with pyroglutamine to prevent mixed species of N-terminal Gln and pyroGlu being generated. This improves the yield of synthesis and ensures a homogeneous chemokine preparation through column manufacture and use. FmocLys(ivDde)-OH is incorporated as residue 75 to facilitate site-specific labelling at this position of the protein (SEQ ID NO: 10). A suitable spacer, such as a PEG spacer, may be incorporated between the ε-amino functionality and the biotin. The biotinylated version comprises, consists essentially of or consists of the amino acid sequence of SEQ ID NO: 11.

Thus, in certain embodiments the invention also relates to a modified chemokine comprising, consisting essentially of or consisting of the amino acid sequence of: X = pyroGlu or Gln
X1 = pyroGlu or Gln
X75 is an amino acid residue that can be biotinylated, such as lysine, ornithine or diaminopropionic acid and optionally is biotinylated, optionally via a spacer molecule such as PEG, optionally K(PEG-Biotin)

A further example of a chemokine of the disclosure containing modifications and specifically adapted is described in detail herein (see Example 4 below). The modified CCL5 (RANTES) corresponds to residues 1 to 68 of the full length mature protein (and lacks the N-terminal signal peptide of 23 amino acids, which is cleaved off) and thus retains the chemokine fold. The single methionine (Met67) within the sequence is mutated to lysine, to mitigate against oxidation of this residue during the chain assembly (SEQ ID NO: 12). This Met to Lys substitution provides a lysine at position 67 which can be modified through biotinylation. FmocLys(ivDde)-OH is incorporated as residue 67 to facilitate site-specific labelling at this position of the protein (SEQ ID NO: 13). The biotinylated version comprises, consists essentially of or consists of the amino acid sequence of SEQ ID NO: 14.

Thus, the disclosure also relates to a modified chemokine comprising, consisting essentially of or consisting of the amino acid sequence of SEQ ID NO: 14: X is an amino acid residue that can be biotinylated, such as lysine, ornithine or diaminopropionic acid and optionally is biotinylated, optionally via a spacer molecule such as PEG (e.g. K(Biotin))

A further example of a chemokine according to the disclosure is containing modifications and specifically adapted for use in the invention is described in detail herein (see Example 3 below). The modified CCL8 (MCP-2) corresponds to residues 1 to 76 of the full length mature protein (and lacks the N-terminal signal peptide of 23 amino acids, which is cleaved off) and thus retains the chemokine fold. The Gln at the N-terminus of the protein is subject to pyroGlu formation under physiological conditions. Thus Gln1 of the sequence is substituted with pyroglutamine to prevent mixed species of N-terminal Gln and pyroGlu being generated (SEQ ID NO: 15). This improves the yield of synthesis and ensures a homogeneous chemokine preparation through column manufacture and use. FmocLys(ivDde)-OH is incorporated as residue 75 to facilitate site-specific labelling at this position of the protein (SEQ ID NO: 16). The naturally occurring lysine at position 75 is modified through biotinylation. A suitable spacer, such as a PEG spacer, may be incorporated between the ε-amino functionality and the biotin (SEQ ID NO: 17):

Thus, the disclosure also relates to a modified chemokine comprising, consisting essentially of or consisting of the amino acid sequence of SEQ ID NO: 17:
X1 = pyroGlu (but may remain as Gln in some embodiments)
X75 = an amino acid residue that can be biotinylated, such as lysine, ornithine or diaminopropionic acid and optionally is biotinylated, optionally via a spacer molecule such as PEG, e.g. K(PEG-Biotin).

The chemokines useful in the various embodiments of the invention can be biotinylated by methods known in the art such as described in WO 00/50088 A2. As indicated above, site-specific labelling of the chemokines of the various embodiments of the invention is preferable, although any labelling technique which does not significantly affect the receptor-binding capacity of the chemokine may be employed. Various site-specifically biotinylated chemokines and native chemokines are available commercially, for instance from Almac, Craigavon, UK. In specific embodiments the chemokines are biotinylated via a spacer group. The spacer may be employed to prevent the biotin group from impacting on the activity of the chemokine, in particular binding of the chemokine to its cognate receptor. Any suitable spacer that facilitates retention of receptor binding properties of the chemokine may be employed in the various embodiments of the invention. Thus, in the specific embodiments described above, spacers other than PEG spacers may be employed as appropriate. In specific embodiments, the spacer is a polyethylene glycol (PEG) spacer. PEG has been shown to be an effective spacer permitting attachment of biotin to the chemokine (which can then be immobilized on the solid support through interaction with streptavidin) without compromising receptor binding capability.

In the context of the disclosure the term "antibody" includes all immunoglobulins or immunoglobulin-like molecules with specific binding affinity for the relevant chemokine receptor (including by way of example and without limitation, IgA, IgD, IgE, IgG and IgM, combinations thereof, and similar molecules produced during an immune response in any vertebrate, for example, in mammals such as humans, goats, rabbits and mice). The term "antibody" also refers to a polypeptide ligand comprising at least a light chain or heavy chain immunoglobulin variable region which specifically recognizes and binds an epitope of an antigen, and it extends to all antibody derivatives and fragments that retain the ability to specifically bind to the relevant chemokine receptor. These derivative and fragments may include Fab fragments, F(ab')₂ fragments, Fv fragments, single chain antibodies, single domain antibodies, Fc fragments etc. The term antibody encompasses antibodies comprised of both heavy and light chains, but also heavy chain (only) antibodies. Exemplary commercially available antibodies which bind to the chemokine receptors of interest are listed in table 1 below. They may or may not be labelled. General reference may be made to "Antibodies a laboratory manual: By E Harlow and D Lane. pp 726. Cold Spring Harbor Laboratory. 1988". Anti-CCR2 antibodies are described for example in WO 2010/021697. Further examples of antibodies include MLN-1202, an anti-CCR2 monoclonal antibody currently undergoing clinical trials (Millennium Pharmaceuticals).

**Table 1. Commercially available fluorophore labelled antibodies against specific chemokine receptors**

| **Antibody** | **Fluorophore** | **Supplier** |
|---|---|---|
| CCR5 | PE | Biolegend |
| CCR3 | PE | Biolegend |
| CCR1 | Alexa Fluor 647 | Biolegend |
| CCR2 | PerCP Cy5.5 | BD Biosciences |
| CCR4 | PerCP Cy5.5 | BD Biosciences |

Solid support materials for immobilizing the binding reagents of the various embodiments of the invention are known in the art. "Solid support" refers to, for example, materials having a rigid or semi-rigid surface or surfaces, and may take the form of beads, resins, gels, microspheres, or other geometric configurations. A useful support material is one that does not activate blood cells so as to make them coagulate or adhere to the support as peripheral whole blood is applied to the device. In certain embodiments, a support treated with an agent to provide it with anti-coagulation properties, in particular a heparinized support is employed. Alternatively, the blood of the patient may be treated with an anti-coagulant such as heparin prior to application to the support. Useful support materials comprise high molecular weight carbohydrates, in particular carbohydrates having a molecular weight of 100 kDa or more, such as agarose, in particulate form, optionally cross-linked, and cellulose. Other preferred support materials are polymers, such as carboxylated polystyrene, and glass. The support of the various embodiments of the invention may be provided in the form of particles or fibres. The support particles may have regular form, such as spheres or beads, or irregular form. They may be porous or non-porous. A preferred average particle size of the support is from 50 µm to 2 mm. In certain embodiments Sepharose™, a cross linked, beaded-form of agarose, is used as column matrix. It is chosen for its optimal distribution capacity and can provide a large available area for affinity binding. Solid supports may be provided in the form of magnetic beads, with the specific binding reagent immobilized on the magnetic beads. Following capture of the CCR2 chemokine receptor expressing cells from the blood, the beads can be removed from the blood with the aid of an appropriate magnetic separator.

Methods for immobilizing binding reagents on a solid support are known in the art. A binding reagent, such as a chemokine, can be immobilized on the support in a direct or indirect manner. Immobilization can be by means of a suitable linker in some embodiments. A preferred method of indirect immobilization of a binding reagent, such as a chemokine, relies upon the interaction between biotin and avidin molecules. "Avidin" or "avidin molecule" refers to any type of protein that specifically binds biotin to the substantial exclusion of other (small) molecules that might be present in a biological sample. Examples of avidin include avidins that are naturally present in egg white, oilseed protein (e.g., soybean meal), and grain (e.g., corn/maize), and streptavidin, which is a protein of bacterial origin. Thus, biotinylation of the binding reagent and use of an avidin molecule such as streptavidin immobilized on the solid support allows reliable attachment of the binding reagent to the solid support according to methods known in the art. Specifically, such a method may comprise providing the binding reagent in biotinylated form, providing a solid support having streptavidin immobilized on its surface, contacting the support with an aqueous solution of the biotinylated binding reagent, and rinsing the support with an aqueous solvent. In addition, binding pair interactions, such as antibody - antigen interactions, may also be utilised for indirect immobilisation of binding reagent onto a support. In such embodiments the support may be derivatised with one member of a binding pair, such as an antibody or fragment or derivative thereof, as defined herein, which has known affinity for a particular peptide sequence or small molecule hapten. Incorporating the other member of the binding pair, such as an antigen, peptide sequence or the hapten onto or into the binding reagent facilitates immobilisation onto a solid support coated with the corresponding antibody or fragment or derivative thereof. Thus, the binding reagent may be modified to include the peptide sequence or hapten into the linear molecule or may be added as a side chain or label. Any suitable antibody-antigen pair may be employed. The antibody fragment or derivative may be any fragment or derivative that retains specific binding affinity for the appropriate antigen. Examples include Fab, F(ab')₂ fragments, scFV, VH domains, single domain antibodies (such as nanobodies), heavy chain antibodies and humanized version of non-human antibodies etc. Other high affinity interactions can be utilised for immobilisation of binding reagents, as long as the binding reagent can be synthesised or derivatised with one of the interacting partners and the solid support synthesised or derivatised with the other interacting partner without loss of binding activity (i.e. binding of the binding reagent to the appropriate chemokine receptor). Immobilization may occur via essentially the same interaction in reverse in some embodiments. Thus, the binding reagent which may be a chemokine for example, may be attached to an antibody as defined herein, and the solid support derivatised with the antigen. The chemokine may be produced as a fusion protein with the antibody.

Alternatively binding reagents, i.e chemokines, can be immobilised directly onto a solid support using bioconjugation techniques established in the field. For example direct immobilisation of proteins onto cyanogen bromide activated solid supports via amino functionalities within the primary sequence of the protein. Alternatively, sulphydryl functionalities within proteins can be used to directly immobilise the protein to alkyl halide derivatised supports or supports containing free thiol functionalities. In further embodiments, proteins containing α-thioester functionalities can be directly immobilised on supports containing 1,2 amino thiol moieties (eg N-terminal cysteine) using the native chemical ligation reaction. Alternatively proteins modified with ketones and aldehydes can be immobilised on solid supports derivatised with hydrazinyl, hydrazide and aminoxy functionalities using hydrazone / oxime bond forming ligation reactions (and vice versa). Alternatively 'Click' chemistry can be used to immobilise proteins onto solid supports, whereby the protein and the support are derivatised with the appropriate mutually reactive chemical functionalities (azides and alkynes). In other embodiments Staudinger ligation chemistry can be used to immobilise appropriately derivatised proteins onto the appropriately derivatised solid supports.

The solid support is contained within or carried by the apheresis column. Thus, by "loaded" is meant that the column carries or contains the solid support in a manner such that (peripheral) blood can flow through the column in contact with the solid support. Thus, the solid support provides a matrix within the column through which blood flows, in continuous fashion in certain embodiments. This permits cells expressing the specific chemokine receptor to be removed from the blood passing through the column, such that blood exiting the column is depleted of the specific chemokine receptor-expressing cells. In specific embodiments, the apheresis column is loaded with a support comprising streptavidin immobilized on the support and one or more biotinylated binding reagents, such as chemokines, bound to the streptavidin on the support. The solid support may be comprised of a high-molecular weight carbohydrate, optionally cross-linked, such as agarose.

As discussed above, the binding reagent is coupled to the solid support. The relative amounts of binding reagent may be controlled to ensure that coupling between the solid support and the binding reagent will be immediate, minimising the risk of binding reagent decoupling from the solid support. Thus, it may be ensured that there is a relative excess of immobilization sites for the binding reagent on the solid support. Alternatively, or additionally, following immobilization of the binding reagent on the solid support, the solid support may be washed to remove any unbound binding reagent.

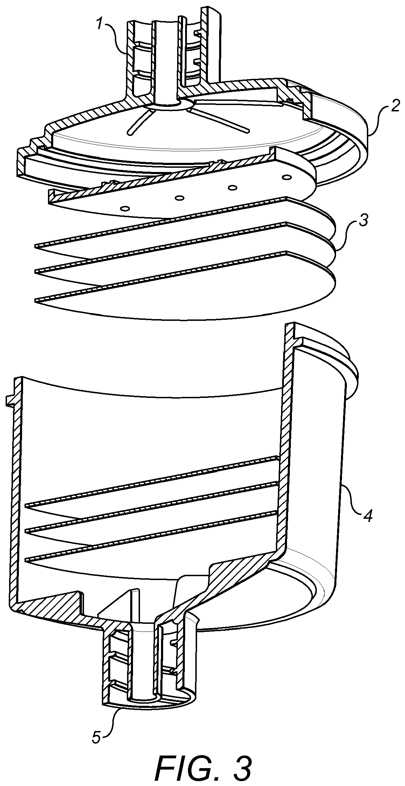

The apheresis column utilised in the various embodiments of the present invention acts as a leukapheresis treatment for conditions associated with metabolic syndrome. The column acts to specifically remove CCR2 -expressing monocytes or leukocytes by exploiting the interaction between CCR2 expressed on the cell surface and a specific binding reagent immobilized on a solid support contained within or carried by the column. The overall column typically comprises, consists of, or consists essentially of three combined components; 1) a housing which contains or carries 2) the solid support and 3) one or more binding reagents (immobilized thereon) which specifically bind one or more chemokine receptors. The housing may be manufactured from any suitable material for clinical use. In certain parts of the disclosure, the housing is composed of a plastic material. The housing includes an in flow site for entry of blood and an out flow site for blood (depleted of target cells) to exit the column. The housing may be designed to maintain a continuous blood flow through the solid support matrix. The housing (as shown for example in Figure 3) may include a top portion which comprises a distribution plate (2) at the inflow site (1) to spread the blood evenly over the entire matrix area. The distribution plate may act as a first safety barrier preventing larger particles flowing through the column and into the patient. However, the distribution plate is not essential and may be removed in some embodiments to decrease the overall resistance in the system. The column may contain one or more safety filter units (3 and 4) placed at the inflow (1) and/or outflow (5) sites of the plastic housing. Such filter units may act to prevent particles larger than blood cells passing in and/or out of the column. The safety filter units may contain a plurality of filters, such as two, three or four filters designed to be a robust barrier and stop all particles larger than blood cells passing through the column. Inclusion of safety filters (3 and 4) at both ends of the column serves to minimize the risk of leakage of particles into the patient, including in the event that the device is incorrectly connected resulting in blood flow in the opposite direction to that intended. The safety filters may comprise of any suitable pore size to prevent particles larger than blood cells from passing through the column, as would be readily understood by one skilled in the art. Suitable filters are commercially available. In specific embodiments, the pore size of the filter(s) is between approximately 60µm and 100µm, more specifically approximately 80 µm. The solid support and binding reagent components are discussed in further detail herein.

The volume of the housing may be varied depending upon the blood volumes intended to pass through the column. Typically, the volume of the housing is between approximately 40ml and 200ml, more specifically 50ml to 150ml or 60ml to 120ml.

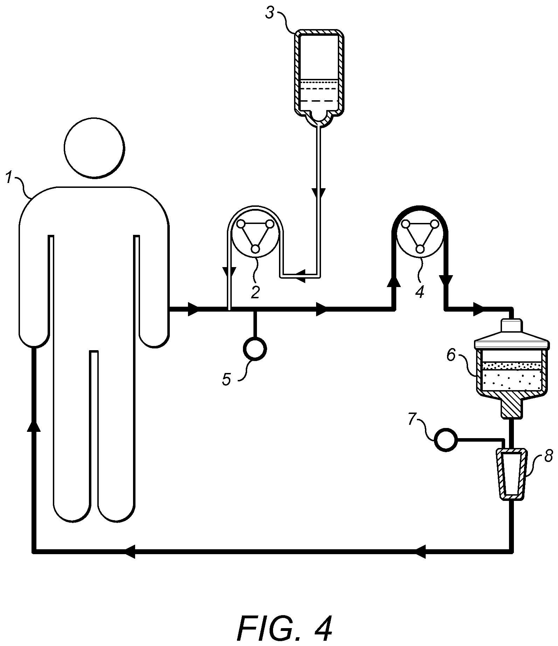

The column is generally applied in the form of an apheresis circuit. In this context, the overall system includes the apheresis column, tubing and an appropriate pump to pump the blood around the circuit. The system is illustrated in figure 4. The patient (1) is connected to the extracorporeal circuit via sterile needles to veins in the right and the left arms. A saline bag (3) is also connected and the saline solution is pumped with a suitable pump (2). Blood is drawn from one arm of the patient through the sterile tubing system by the blood pump (4) and passed through the column (6) and back to the patient. The tubing system may be connected to the column via any suitable coupling, such as standard dialysis luer-lock couplings. The couplings on the column may be colour-coded for correct assembly. For example, red tubing for inflow to the red column top and blue tubing for outflow back to the patient. An air detector (8) may be present in the circuit. Inlet pressure (5) and/or Pven sensors (7) may additionally be employed to monitor the pressure in the circuit.

An apheresis pump, such as the 4008 ADS pump manufactured by Fresenius Medical Care or the Adamonitor pump, may monitor the patient's inflow and outflow. The pump may also monitor the pressure in the extracorporeal circulation. The pump may be able to discriminate air by a bubble catcher and air detector. A clot catcher filter may be positioned inside the bubble catcher. The pump may also incorporate an optical detector to distinguish between light, e.g. saline solution or air present in the tubing system and dark e.g. blood present in the tubing system.

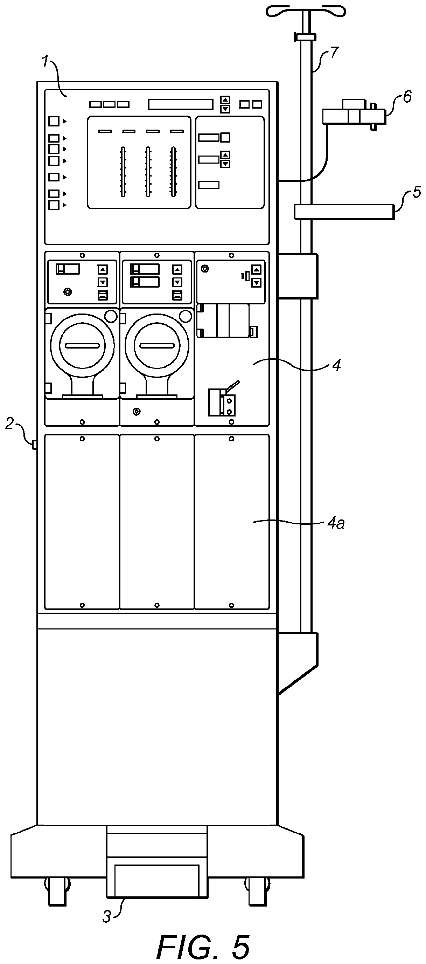

A schematic diagram of a suitable pump, showing the air detector and optical filter is shown in figure 5. If the pump system detects air bubbles and optical fluctuations or if extracorporeal pressure values are out of the set range, then the pump may stop immediately. Alternatively or additionally a visual/ audible alarm may be emitted.

The various embodiments of the invention and the disclosure will now be described in more detail by reference to the following embodiments and examples:

### DESCRIPTION OF THE FIGURES

FIG. 1a, 1b & 1c - the binding of biotinylized MCP-1 by CD4+, CD8+ T-cells and CD14+ monocytes respectively, obtained from peripheral blood of a healthy donor.
FIG. 2a - binding of MCP-1 to monocytes (dashed line) in peripheral blood taken from IBD patients. The graph represents a summary of four tests.
FIG. 2b - binding of CCR2-antibody to monocytes (line) in peripheral blood taken from IBD patients. The graph represents a summary of four tests.
FIG. 3 - The plastic house and top showing the distribution plate (2) and safety filter units (3 and 4).
FIG. 4 - The overall leukapheresis system.
FIG. 5 - The pump with air detector and optical detector (4).
FIG. 6a - Results of in vitro depletion tests performed on the bMCP-1 coupled matrix showing ability to eliminate CCR2-expressing cells from blood from three healthy donors.
FIG. 6b - Results of in vitro depletion tests performed on the biotinylated RANTES coupled matrix showing ability to eliminate chemokine receptor-expressing cells from peripheral blood taken from a healthy donor.
FIG. 7 - Sequence and biotinylation, via a spacer group, of mature protein MCP-1 derivative containing Gln to pyroGlu modification
FIG. 8 - Sequence and biotinylation, via a spacer group, of mature protein MCP-1 derivative containing Gln to pyroGlu modification and Met to Norleu substitution
FIG. 9 - Sequence and biotinylation, via a spacer group, of truncated MCP-1 derivative containing Met to Norleu substitution
FIG. 10 - Alignment of MCP-1 and MCP-5 amino acid sequences
FIG. 11 - Sequence and biotinylation, via a spacer group, of (C-terminal) truncated MCP-5 derivative containing Ile to Lys modification
FIG. 12 - Sequence and biotinylation, of RANTES derivative

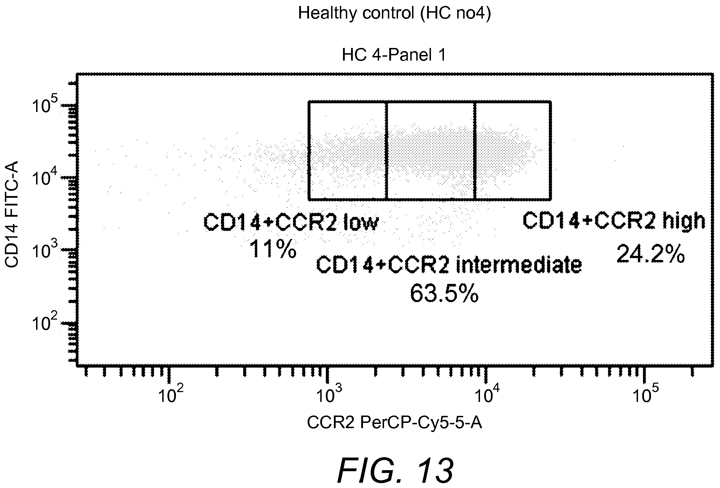

FIG. 13 - Example of gating criteria for CCR2-expressing monocytes
FIG. 14 - Expression of CCR2 expressing monocytes in 9 patients with diabetes mellitus (DM) and in 20 healthy controls (HC). Blood from patients with DM and healthy controls was analysed for the expression of various chemokine receptors by flow cytometry. The monocytes were characterized as CD14 positive cells.
FIG. 15 - Binding of the chemokine bMCP1 (CCL2) to monocytes from 3 patients with Diabetes Mellitus and in 20 healthy controls (HC). Blood from 3 patient with DM was incubated with bMCP1 and analysed with flow cytometry. The monocytes were characterized as CD14 positive cells.
FIG. 16 - Depletion of CCR2 expressing monocytes with Sepharose Streptavidin-matrix conjugated with bMCP1. Blood cells from a patient with DM were incubated with bMCP1-Sepharose Streptavidin-matrix. Unbound cells were retrieved by washing the matrix with Phosphate Buffer Saline. The cells (After Depletion) were then analysed with flow cytometry and compared with cells that had not been incubated with bMCP1-matrix (Before Depletion).
FIG. 17 - Expression of CCR2 expressing B cells in seven patients with adiposis dolorosa (AD) and in 20 healthy controls (HC). Blood from patients with AD and healthy controls was analysed for the expression of various chemokine receptors by flow cytometry. The B cells were characterized as CD19 positive cells.
FIG. 18 - Binding of the chemokine bMCP1 (CCL2) to B cells from seven patient with Adiposis Dolorosa . Blood from a patient with AD was incubated with bMCP1 and analysed with flow cytometry. The B cells were characterized as CD19 positive cells.
FIG. 19 - Depletion of CCR2 expressing B cells with Sepharose Streptavidin-matrix conjugated with bMCP1. Blood cells from a patient with AD were incubated with bMCP1-Sepharose Streptavidin-matrix. Unbound cells were retrieved by washing the matrix with Phosphate Buffer Saline. The cells (After Depletion) were then analysed with flow cytometry and compared with cells that had not been incubated with bMCP1-matrix (Before Depletion).
FIG. 20 - Increased frequency of CCR1 expressing monocytes in 4 patients with Adiposis dolorosa compared to healthy controls.
FIG. 21 - Depletion of CCR1 expressing moncoytes with SepharoseStrepavidin-matrix-bRANTES. Blood cells from a healthy control were incubated with biotinylated chemokine-Sepharose Streptavidin-matrix. Unbound cells were retrieved by washing the matrix. The cells (After Depletion) were then analysed with flow cytometry and compared with cells that had not been incubated with bchemokine-matrix (Before Depletion).
FIG. 22 - Increased frequency of CCR4 expressing T cells in four patients with type 2 Diabetes.
FIG. 23 - Increased frequency of CCR5 expressing T cells in four patients with type 2 Diabetes.

### DESCRIPTION OF PREFERRED EMBODIMENTS

It has been shown (Jianli Niu and Pappachan E. Kolattakudy.Clinical Science (2009) 117, 95-109) that CCR2 expression on monocytes is elevated in diabetic patients. Transgenic mice with an adipose-tissue-specific expression of MCP-1 have macrophage infiltration into adipose tissue, increased hepatic triacylglycerol content and insulin resistance. MCP-1-knockout mice fed a high□fat diet have a drastically reduced macrophage accumulation into adipose tissue and hepatic steatosis when compared with high-fat□fed wild□type mice. Inhibition of MCPD□1 function by the acute expression of a dominant negative mutant of MCP-1 ameliorated insulin resistance in db/db mice and in high-fat-fed wild-type mice. Moreover, increased level of serum MCP-1 correlates with markers of the metabolic syndrome, including obesity, insulin resistance, Type 2 diabetes, hypertension and increased serum triacylglyerol concentrations. Monocyte chemoattractant protein-1 (MCP-1) is a major chemoattractant for monocytes and memory T cells by means of their binding to its specific cell-surface receptor, CC-chemokine receptor-2 (CCR2). On this basis, the inventors have selected CCR2 expressing cells as a target for treatment of conditions associated with metabolic syndrome.

It is shown herein that MCP-1 can be used to reduce CCR2-expressing monocyte levels in diabetic subjects.

### Materials and methods

*Isolation of peripheral blood leukocytes.* Heparinized peripheral blood from healthy blood donors or inflammatory bowel disease (IBD) patients was fixed with 4% paraformaldehyde for 4 minutes, hemolyzed for 15 minutes with a 0.83 % ammonium chloride solution and washed twice in FACS buffer to obtain a suspension of blood leukocytes.

*Chemokines.* The leukocytes were incubated for 30 min in the dark at 4 °C with biotinylated and Alexa647 Fluor® labeled MCP-1 (in concentrations 10 ng/µL and 50 ng/µL). The cells were then washed with FACS-buffer and analyzed by flow cytometry. All chemokines used in the Examples were provided by Almac Sciences Scotland Ltd, Edinburgh, Scotland.

*Flow cytometry assay.* The flow cytometry assay was performed on a two laser FACS Calibur cytometer (BD Immunocytometry systems, San Jose, Ca, USA). Ten thousand cells were counted and analysed in each sample. For data analyses, Cell Quest Pro software from Becton Dickinson was used.

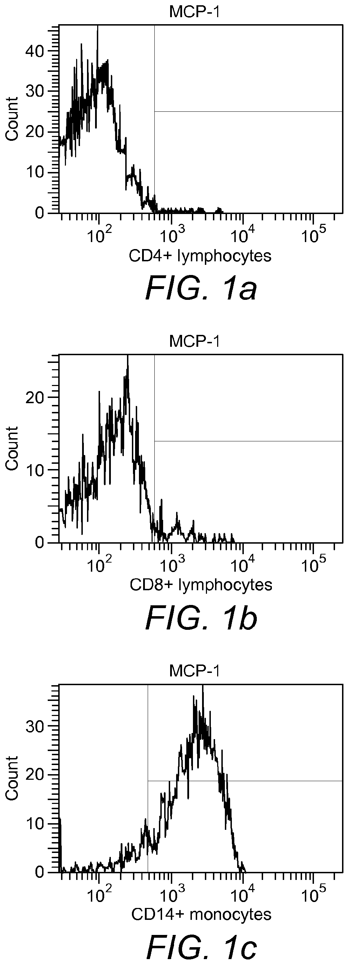

**EXAMPLE 1-** *Binding of monocytes to MCP-1.* In the experiment with biotinylated MCP-1 it was found that about 90% of the monocytes obtained from peripheral blood of healthy donors had bound to the cytokine after 30 min of incubation (Fig. 1a), whereas CD4+ and CD8+ lymphocytes had not bound (Fig. 1b and 1c).

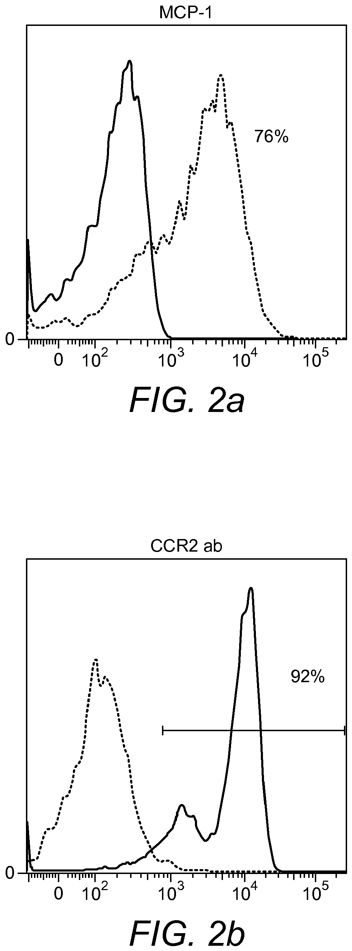

**EXAMPLE 2** - Monocytes were investigated for their expression of CCR2 (FIG. 2b) and their ability to bind MCP-1 (FIG. 2a). CCR2 expression was noted an all monocytes with the majority of monocytes expressing high levels, using an anti-CCR2 antibody (FIG. 2b). The MCP-1 binding to monocytes shown in FIG. 2a corresponds to the CCR2^{hi} expressing population shown in FIG. 2b. Thus, MCP-1 binds favourably to CCR2^{hi} expressing cells.

### EXAMPLE 3 - Tailored leukapheresis

### COLUMN DESIGN AND PROPERTIES

### Introduction

Apheresis is an established treatment used for depletion of blood components, such as antibodies, low-density lipoproteins (LDL) and blood cells. Leukapheresis is the apheresis treatment used for removal of white blood cells, leukocytes. The patient is connected to an extracorporeal blood circulating system; the blood is drawn from a vein in one arm, passed through a column device and returned into the other arm of the patient. Side effects of leukapheresis treatments are varying from mild events like headache, dizziness, hypotension, palpitation and flush seen in 0.1 to 5% of treated patients.

### The column

The column is intended to be used as a leukapheresis treatment for a condition associated with metabolic syndrome. It will specifically remove CCR2, and CCR1, CCR3, CCR4 or CCR5-expressing leukocytes as reference/comparison examples, in particular monocytes, through the use of a binding reagent, more specifically an MCP-1, and MCP-2, MCP-3, MCP-4, MCP-5 and/or RANTES containing resin as reference/comparison examples, exploiting the CCR2, CCR1, CCR3, CCR4 or CCR5-chemokine interaction. The column consists of three combined components, the plastic house, the streptavidin (SA) Sepharose™ BigBeads matrix and one or more of biotinylated MCP-1, MCP-2, MCP-3, MCP-4, MCP-5 and RANTES bound to the matrix. The treatment is conducted using the same techniques as a standard apheresis procedure.

### The plastic house (FIG. 3)

The plastic house, designed to keep a continuous blood flow through the matrix, consists of a transparent body and red-coloured top. The top has a distribution plate (2) at the inflow site (1) to spread the blood evenly over the entire matrix area. The plate is the first safety barrier preventing larger particles flowing through the column and into the patient. Safety filter units (3 and 4) are placed at the inflow (1) and outflow (5) sites of the plastic housing. The safety filter unit contains three filters designed to be a robust barrier and stop all particles larger than blood cells passing through the column. The plastic housing design is shown in Figure 3. The design with safety filters (3 and 4) at both ends of the column device will minimize the risk of leakage of particles into the patient, including in the event that the device is placed up side down with the blood flow in the opposite direction to that anticipated.

### Streptavidin Sepharose™ BigBeads

The second component in the device is the affinity matrix called streptavidin Sepharose™ BigBeads (Sepharose™ GE Healthcare, Sweden). Sepharose™ is a cross linked, beaded-form of agarose, which is a polysaccharide extracted from seaweed. Sepharose™ and agarose are commonly used as column matrices in biomedical affinity techniques. It is chosen for its optimal distribution capacity and can provide a large available area for affinity binding.

### Binding reagent

Coupled to the matrix is the third component of the device, one or more binding reagents that bind specifically to CCR2, CCR1, CCR3, CCR4 or CCR5. One or more hemokines selected from the group consisting of: MCP-1, MCP-2, MCP-3, MCP-4, MCP-5, RANTES, CCL17 (TARC) and CCL22 (MDC) may be employed. These peptides may be synthetic, engineered versions of the human chemokine, which are truncated and biotinylated, but retain binding activity to the CCR2, CCR1, CCR3, CCR4 or CCR5 receptor. By biotinylating the engineered chemokine, it is able to bind to the streptavidin molecules in the Sepharose™ matrix. The biotin-streptavidin binding is known be one of the strongest biological interactions with a Kd in the order of 4 x 10⁻¹⁴ M. The calculated ratio of streptavidin:biotin binding sites in the column is 10:1. Therefore, the coupling between the matrix and chemokine will be immediate, minimising the risk of chemokine decoupling from the matrix.

### The apheresis system

To conduct the leukapheresis the following components are needed; the column, tubing system, and a 4008 ADS pump (Fresenius Medical Care).

### The circuit

The system is illustrated in Figure 4. The patient (1) is connected to the extracorporeal circuit via sterile Venflon needles to veins in the right and the left arms. A saline bag (3) is also connected and the saline solution is pumped with an ACD pump (2). Blood is drawn from one arm of the patient through the sterile tubing system by the blood pump (4) and passed through the column (6) and back to the patient. The tubing system is connected to the column via standard dialysis luer-lock couplings. The couplings on the column are colour-coded for correct assembly; red tubing for inflow to the red column top and blue tubing for outflow back to the patient. An air detector (8) is present. Inlet pressure(5) and Pven sensors (7) are employed to monitor the pressure in the circuit.

### The 4008 ADS pump

An apheresis pump, from Fresenius Medical Care, monitors the patient's inflow and outflow, the pressure in the extracorporeal circulation and can discriminate air by a bubble catcher and air detector. A clot catcher filter is placed inside the bubble catcher. The pump also has an optical detector to distinguish between light, e.g. saline solution or air present in the tubing system and dark e.g. blood present in the tubing system.

A schematic diagram of the pump, showing the air detector and optical filter is shown in Figure 5. If the pump system detects air bubbles and optical fluctuations or if extracorporeal pressure values are out of the set range, then the pump stops immediately and a visual/ audible alarm are emitted.

### Legend for FIG. 5:

- 1.: Monitor
- 2.: Holder for waste bag
- 3.: Modules (left to right - Blood pump, ACD pump, Air detector)
- 4.: Reserve places for further modules
- 5.: Absorber holder
- 6.: Drip detector
- 7.: IV pole

### Preparation of the patient

The patient will be administered anticoagulants prior to each treatment session. A sterile saline solution with 5000 IE Heparin will be used for priming the extracorporeal system, thereafter a bolus injection with 4000 IE Heparin will be added into the circuit at the start of each treatment session.

### Leukapheresis time and flow rate

The apheresis system should be operated at a flow rate of 30-60 mL/min. A treatment is finalised after 1800mL of blood has been circulated.

### Storage conditions

The column devices should be stored between 1 and 25°C avoiding freezing and more elevated temperatures. Stability data > 3 months indicate no difference in functionality over time or by temperature (room temperature and refrigerated). The columns will be kept in refrigerated conditions until use. Mechanical damage as those resulting from violent vibrations and trauma should be avoided. Column stored outside of these recommendations should not be used.

### Transport conditions

The column devices will be transported under refrigerated condition, avoiding freezing and more elevated temperatures. Mechanical damage such as those resulting from violent vibrations and trauma should be avoided.

### In-vitro depletion of target cell populations

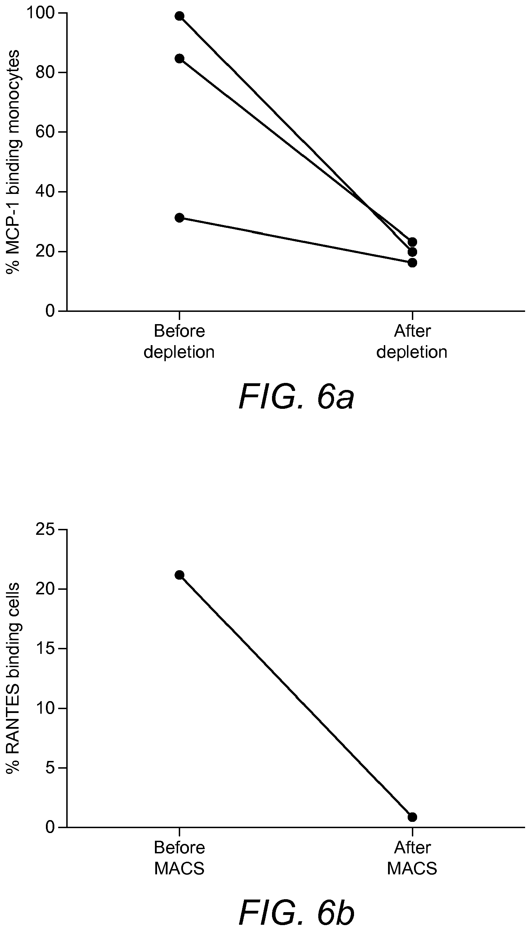

To investigate the ability to eliminate CCR2-expressing cells, in vitro tests have been performed on the bMCP-1 coupled matrix. Blood was collected from blood donors and passed through the column device containing bMCP-1 coupled matrix. Blood samples were taken before and after column passage and analyzed by flow cytometry (FACS) for the depletion of CCR2-expressing cells.
The results demonstrate significant depletion of the target population CCR2-expressing monocytes post matrix perfusion. Depletion tests were performed on blood from three healthy donors. The results are shown in Figure 6a.
The in-vitro results demonstrate a specific reduction of up to 80% of the CCR2 -expressing cells by the column. Notably, individuals with fewer CCR2 expressing cells initially achieved lower depletion. The remaining levels of monocytes were around 20-30% in each case, irrespective of the starting point. Non-CCR2 -expressing cells remained unaffected (data not shown).

To investigate the ability to eliminate CCR1 (as reference/comparison example), 3 and 5-expressing cells, in vitro tests have been performed on the biotinylated RANTES (as reference/comparison example) coupled matrix. Blood was collected from blood donors and passed through the column device containing biotinylated RANTES coupled matrix. Blood samples were taken before and after column passage and analyzed by flow cytometry (FACS) for the depletion of CCR1, 3 or 5-expressing cells.

The RANTES molecule was synthesized by Almac. The amino acid sequence of the biotinylated RANTES molecule is set forth as SEQ ID NO: 8:

This molecule has the naturally occurring methionine at postion 67 replaced with lysine to facilitate biotinylation at position 67.

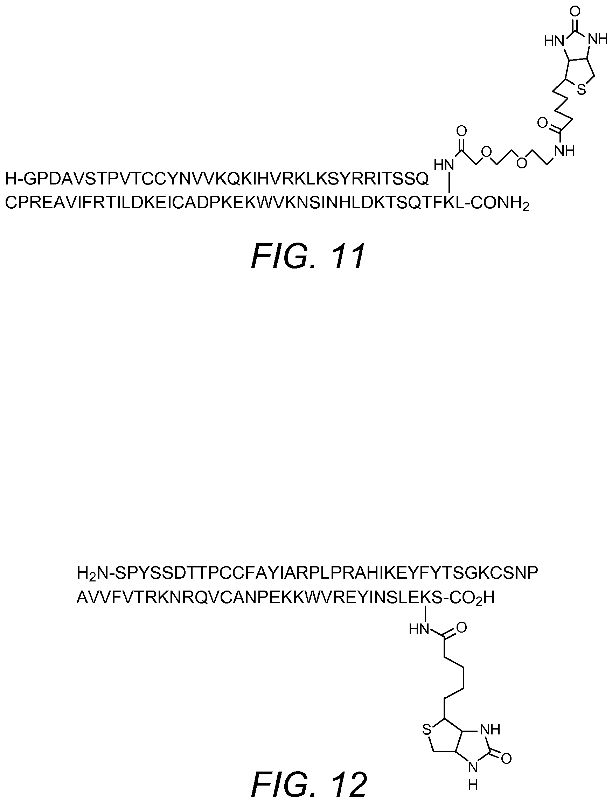

The side-chain of Lys 67 was directly biotinylated to given the protein primary structure shown in figure 12. The protein was folded and disulphide bonds formed between the first and third cysteine in the sequence and between the 2nd and 4th cysteines.

The results demonstrate significant depletion of the target population chemokine receptor-expressing cells post matrix perfusion. Depletion tests were performed on blood from a healthy donor. The results are shown in Figure 6b.
The in-vitro results demonstrate a specific reduction of around 20% of the chemokine receptor-expressing cells by the column. Non- CCR1, 3 and 5-expressing cells remained unaffected (data not shown).

### EXAMPLE 4 - MCP1 DERIVATIVES

MCP-1 has been produced with residue 75 as the site of biotinylation on the chemokine (numbering based upon the mature protein having the amino acid sequence of SEQ ID NO: 2). Biotinylation permits immobilization of MCP-1 on a solid support (via a biotin-avidin interaction). The basic amino acid sequence of MCP-1, including a 23 amino acid leader sequence is set forth as SEQ ID NO: 1,

The amino acid sequence of the mature protein is set forth as SEQ ID NO: 2, X = Met or Norleucine
The inventors have determined that chemokines may display improved binding properties where the chemokine is biotinylated via a spacer group. The spacer may prevent the biotin group from impacting on the binding affinity of the chemokine.

Thus, MCP-1 derivatised at the ε-amino side chain functionality of Lys75 with PEG-Biotin (TFA salt) will be synthesised. The PEG spacer will be 3,6, - dioxoaminooctanoic acid. The Gln at the N-terminus of the proteins is subject to pyroGlu formation under physiological conditions. Thus the first glutamine (Gln1) of the sequence will be substituted with pyroglutamine. The molecule will be synthesised as a C-terminal amide (via synthesis on an amide linker). The molecule is shown schematically in Figure 7.

A biotinMCP-1 Met to Nleu analogue will also be synthesised. The single methionine within the sequence will be altered to Norleucine, to mitigate against oxidation of this residue during the chain assembly and improve stability of the final product. This molecule is shown schematically in Figure 8, see also SEQ ID NO: 2.

Once synthesised, the activity of the various biotinMCP-1 derivatives will be determined in cell-based assays. In particular, agonist and antagonist properties will be determined in aequorin functional cell-based assay on human CCR2 receptor. The relevant molecules have been synthesised, see Example 7 below.

### EXAMPLE 5 - SYNTHESIS OF A CCR2 ANTAGONIST BIOTINMCP-1 WHICH BINDS TO THE RECEPTOR WITHOUT ACTIVATION

Antagonist Activity (J-H Gong and I. Clark-Lewis, J. Exp. Med., 1995, 181, 63) has been shown for an MCP-1 derivative truncated at the N-terminus. In particular, deletion of residues 1-8, results in binding to CCR2 with Kd 8.3 nM. This protein was unable to cause chemotaxis of CCR2 positive cells. (inhibition of chemotaxis IC50 20nM)

The amino acid sequence of the truncated version is set forth as SEQ ID NO: 3:

A derivative of this truncated version will be synthesised comprising residues 9 to 76 of the mature protein (MCP-1 9-76) with Met64 to Nleu substitution and derivatised at the ε-amino side chain functionality of Lys75 with PEG-Biotin (TFA salt). This molecule is shown schematically in Figure 9. The PEG spacer will be 3,6, - dioxoaminooctanoic acid.

Once synthesised, the activity of the various biotinMCP-1 derivatives will be determined in cell-based assays. In particular, agonist and antagonist properties will be determined in aequorin functional cell-based assay on human CCR2 receptor.

### EXAMPLE 6 - DEMONSTRATE REMOVAL OF CCR2 EXPRESSING CELLS USING AN ALTERNATIVE CHEMOKINE LIGAND TO MCP-1 (Comparison/reference experiment)

CCR2 also binds chemokines MCP-2, MCP-3, MCP-4, MCP-5, and HCC-4 in addition to MCP-1. MCP-5 only binds CCR2 and should be selective in its removal of CCR2 expressing cells. MCP5 is a mouse chemokine shown to chemotact human CCR2 cells with EC50 < 3 nM.

The full length amino acid sequence, including the signal peptide, is set forth as SEQ ID NO: 4

The amino acid sequence of N-terminal processed MCP-5 chemokine is 82 amino acids long and is set forth as SEQ ID NO: 5

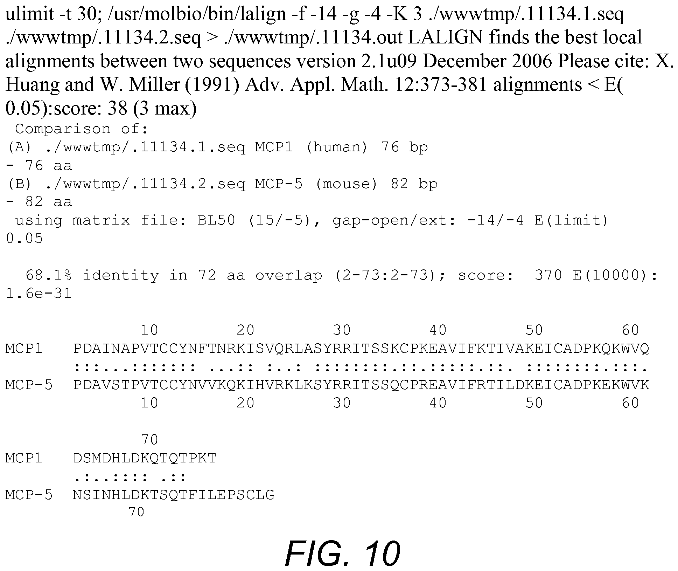

An amino acid sequence alignment suggests that MCP-5 has a C-terminal extension when compared to the amino acid sequence of MCP-1. The results of this alignment are shown in figure 10. On this basis a C-terminal truncated version of MCP-5 will be synthesised. This truncated version will comprise MCP-5 residues 1-76, set forth as SEQ ID NO: 6:

In the truncated version, Ile75 to be substituted with Lys, set forth as SEQ ID NO: 7:

Following substitution, the substituted version will be biotinylated at position 75, a lysine or other suitable residue such as ornithine or diaminopropanoic acid via A PEG spacer (3,6, - dioxoaminooctanoic acid). The protein will be synthesised on an amide linker to yield a C-terminal amide derivative. This molecule is shown schematically in Figure 11.

Once synthesised, the activity of the various biotinMCP-5 derivatives will be determined in cell-based assays. In particular, agonist and antagonist properties will be determined in aequorin functional cell-based assay on human CCR2 receptor.

### EXAMPLES 7 to 9

### SYNTHESIS OF ADDITIONAL CHEMOKINES

### General Protocols

### Assembly:

Chemical synthesis of chemokines was performed using standard Fmoc solid phase peptides synthesis (SPPS) techniques on an ABI 433 peptide synthesiser. DIC (0.5 M in DMF) and OxymaPure (0.5 M in DMF) were used for activation, acetic anhydride (0.5 M in DMF) for capping, and 20 % piperidine in DMF for Fmoc deprotection. Rink Amide resin was utilised for the generation of C-terminal amide chemokines and Wang resin for C-terminal acid chemokines. After assembly, the resin was washed with DMF and DCM and then dried *in vacuo.*

### Removal of Dde Protection:

The Dde protecting group was removed by treatment of resin with a solution of 2.5% hydrazine in DMF (200ml) over a 2 hour period. The resin was then washed with DMF.

### Labelling steps:

### 1. Couple Fmoc-8-amino-3,6-dioctanoic acid (PEG)

Resin was swollen in DMF and then a solution of Fmoc-8-amino-3,6-dioctanoic acid (0.38g, 1mmol), DIC solution (2ml, 0.5 M in DMF) and OxymaPure solution (2ml, 0.5 M in DMF) was added. The mixture was sonicated for 3 hours and then washed with DMF.

### 2. Capping

The resin was capped with acetic anhydride solution (0.5 M in DMF, 10ml) for 5 minutes and then washed with DMF.

### 3. Fmoc deprotection

Fmoc deprotection was carried out by treatment with 20% piperidine in DMF solution (2 x 50ml) for 15 minutes each. The resin was washed with DMF.

### 4. Couple Biotin-OSu

A solution of Biotin-OSu (341mg, 1mmol) and DIPEA (348µl) in DMF (10ml) was added to the resin and the mixture was sonicated for 3 hours. The resin was washed thoroughly with DMF and DCM then dried *in vacuo.*

### Cleavage:

Dry resin was treated with TFA (10 ml) containing a scavenger cocktail consisting of TIS (500 µl), thioanisole (500 µl), water (500 µl), DMS (500 µl), EDT (250 µl), NH₄I (500 µg) and phenol (500 µg) and the mixture was stirred at room temperature for 5 hours. The solution was filtered into cold ether and the resin rinsed with TFA. The precipitated peptide was centrifuged, washed with ether, centrifuged and lyophilised.

### Purification Protocol:

The crude peptide was purified by reverse phase HPLC (RP-HPLC) using a Jupiter C18, 250 x 21 mm column, 9 ml/min, eluting with an optimised gradient [Buffer A: water containing 0.1% TFA, Buffer B: acetonitrile containing 0.1% TFA].

### Folding Protocol:

Pure peptide (10mg) was dissolved into 6M GnHCI (16 ml) and then rapidly diluted to 2M GnHCI concentration by the addition of 50mM TRIS pH 8.5 (84 ml) containing 0.3mM GSSG and 3mM GSH. The mixture was stirred at room temperature for 24 hours and then analysed by RP-HPLC (Jupiter C18, 250 x 4.6 mm column, 10-60% B over 30 minutes. Purification by RP-HPLC using an optimised gradient afforded the desired product.

### EXAMPLE 7 - biotinMCP-1 (CCL2)

Target Molecule: MCP-1 derivatised at the ε-amino side chain functionality of Lys(75) with PEG-Biotin (TFA salt)

Modifications: Human MCP-1 corresponding to residues 1-76, is initially expressed as 99 amino acids comprising the chemokine fold, and a 23 amino acid signal peptide which is cleaved off. The Gln at the N-terminus of the protein is subject to pyroGlu formation under physiological conditions. Thus Gln1 of the sequence was substituted with pyroglutamine to prevent mixed species of N-terminal Gln and pyroGlu being generated. This improves the yield of synthesis and ensures a homogeneous chemokine preparation through column manufacture and use. The naturally occurring lysine at position 75 was modified through biotinylation on the resin. A PEG spacer was incorporated between the ε-amino functionality and the biotin.

The linear amino acid sequence (SEQ ID NO:9) is shown, prior to attachment of the PEG spacer and biotin molecules at amino acid 75 (K): X = pyroGlu or Gln

The engineered MCP-1 sequence was assembled on a solid support (Rink Amide resin), using Fmoc protocols for solid-phase peptide synthesis as described in the general protocols section:
X1 = pyroGlu or Gln
X75 = K(ivDde)

FmocLys(ivDde)-OH was incorporated as residue 75 to facilitate site-specific labelling at this position of the protein. Subsequent removal of the ivDde protecting group, followed by coupling of the PEG spacer and Biotin, was carried out as described in the general protocol section. Cleavage, purification and folding protocols were carried out as described to furnish the desired active chemokine.
X1 = pyroGlu or Gln
X75 is an amino acid residue that can be biotinylated, such as lysine, ornithine or diaminopropionic acid and optionally is biotinylated, optionally via a spacer molecule such as PEG, optionally K(PEG-Biotin)

Electrospray ionisation with tandem mass spectrometry (ESI-TOF-MS) data of purified folded biotinMCP-1: obtained = 9032.8 Da; expected 9034.4 Da.

### Functional Assay Data:

biotinMCP-1 was tested for agonist activity in an Aequorin assay against hCCR2b, (Euroscreen) and an EC50 value of 9.6nM was reported. c.f. EC50 for recombinant native MCP-1 is 3.1nM.

### EXAMPLE 8 - biotinRANTES (CCL5), reference/comparison example

Target Molecule: RANTES derivatised at the ε-amino side chain functionality of Lys(67) with Biotin (TFA salt)

Modifications: Human RANTES corresponding to residues 1-68, is initially expressed as 91 amino acids comprising the chemokine fold, and a 23 amino acid signal peptide which is cleaved off. The single methionine (Met67) within the sequence was mutated to lysine, to mitigate against oxidation of this residue during the chain assembly, which was observed during the synthesis of the natural sequence derivative. This Met to Lys substitution provided a lysine at position 67 which was modified through biotinylation on the resin.

The linear amino acid sequence (SEQ ID NO: 12) is shown, prior to attachment of the biotin molecule at amino acid 67 (K):

The engineered RANTES sequence was assembled on a solid support (Wang resin), using Fmoc protocols for solid-phase peptide synthesis as described in the general protocols section: X is K(ivDde)

FmocLys(ivDde)-OH was incorporated as residue 67 to facilitate site-specific labelling at this position of the protein (SEQ ID NO: 13). Subsequent removal of the ivDde protecting group, followed by coupling of the Biotin, was carried out as described in the general protocol section. Cleavage, purification and folding protocols were carried out as described to furnish the desired active chemokine (SEQ ID NO: 14). X is an amino acid residue that can be biotinylated, such as lysine, ornithine or diaminopropionic acid and optionally is biotinylated, optionally via a spacer molecule such as PEG (e.g. K(Biotin))

Electrospray ionisation with tandem mass spectrometry (ESI-TOF-MS) data of purified folded biotinRANTES: obtained = 8068.9 Da; expected 8070.2 Da.

### Functional Assay Data:

BiotinRANTES was tested for agonist activity in an Aequorin assay against hCCR5, (Euroscreen) and an EC50 value of 0.5nM was reported.

### EXAMPLE 9 - biotinMCP-2 (CCL8), reference/comparison example

Target Molecule: MCP-2 derivatised at the ε-amino side chain functionality of Lys(75) with PEG-Biotin (TFA salt)

Modifications: Human MCP-2 corresponding to residues 1-76, is initially expressed as 99 amino acids comprising the chemokine fold, and a 23 amino acid signal peptide which is cleaved off. The Gln at the N-terminus of the protein is subject to pyroGlu formation under physiological conditions. Thus Gln1 of the sequence was substituted with pyroglutamine to prevent mixed species of N-terminal Gln and pyroGlu being generated. This improves the yield of synthesis and ensures a homogeneous chemokine preparation through column manufacture and use. The naturally occurring lysine at position 75 was modified through biotinylation on the resin. A PEG spacer was incorporated between the ε-amino functionality and the biotin.

The linear amino acid sequence (SEQ ID NO: 15) is shown, prior to attachment of the PEG spacer and biotin molecules at amino acid 75 (K): X = pyroGlu or Gln

The engineered MCP-2 sequence was assembled on a solid support (Rink Amide resin), using Fmoc protocols for solid-phase peptide synthesis as described in the general protocols section:
X1 = pyroGlu or Gln
X75 = K(ivDde)

FmocLys(ivDde)-OH was incorporated as residue 75 to facilitate site-specific labelling at this position of the protein (SEQ ID NO: 16). Subsequent removal of the ivDde protecting group, followed by coupling of the PEG spacer and Biotin, was carried out as described in the general protocol section. Cleavage, purification and folding protocols were carried out as described to furnish the desired active chemokine (SEQ ID NO: 17):
X1 = pyroGlu or Gln
X75 = an amino acid residue that can be biotinylated, such as lysine, ornithine or diaminopropionic acid and optionally is biotinylated, optionally via a spacer molecule such as PEG, e.g. K(PEG-Biotin).

Electrospray ionisation with tandem mass spectrometry (ESI-TOF-MS) data of purified folded biotinMCP-2: obtained = 9263.6 Da; expected 9263.8 Da.

### Functional Assay Data:

biotinMCP-2 was tested for activity in an Aequorin assay against hCCR2b, (Euroscreen) and was shown to be a partial agonist with an EC50 value of 50.9nM. c.f. EC50 for recombinant native MCP-2 is 23.5nM (partial agonist).

### EXAMPLE 10 - Diagnosis and Treatment of Diabetes Mellitus (DM)

### Materials and methods

### 1. Flow cytometric analysis of peripheral blood

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH₄Cl, pH 7.5) for 15 min at 37°C. The cells were washed in PBS with 2% Bovine Growth Serum, incubated with 10% human serum for 15min at room temperature (RT) and stained with antibodies (Table 2) at 4°C for 30min. The cells were analysed by flow cytometry on a FACS Canto flow cytometer with the FACSDiva software (BD Biosciences).

**Table 2. List of antibodies for flow cytometric analysis.**

| **Antibody** | **Fluorophore** | **Supplier** |
|---|---|---|
| CD14 | FITC | Beckman Coulter |
| CCR5 | PE | Biolegend |
| CCR3 | PE | Biolegend |
| Streptavidin | PE, APC | Biolegend |
| CCR2 | PerCP Cy5.5 | Biolegend |
| CD3 | V450 | BD Biosciences |
| CD19 | V500 | BD Biosciences |
| CD16 | PE Cy7 | BD Biosciences |
| CCR4 | PerCP Cy5.5 | BD Biosciences |

### 2. Chemokine Binding test

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH4CI, pH 7.5) for 15 min at 37°C. The cells were washed in PBS with 2% Bovine Growth Serum, incubated with 10% human serum 15min at room temperature (RT) and stained with cell specific antibodies together with biotinylated chemokine (1 µM) or the corresponding chemokine receptor antibody at 4°C for 30min (Table 2). The biotinylated chemokine was detected via the interaction between biotin and a fluorophore conjugated Streptavidin. The samples were analysed by flow cytometry on a FACS Canto flow cytometer with the FACSDiva software (BD Biosciences).

### 3. Cell depletion by matrix conjugated with biotinylated chemokine

Cells were prepared from peripheral blood (section 1). 1 mL Sepharose BigBeads matrix conjugated with 0.4mg/mL Streptavidin (GE Healthcare) was washed in 50 mL PBS and added to a 5 mL polystyrene tube (BD Falcon™). Biotinylated chemokine (1µM) was added to the tube and incubated for 20 min at RT to enable immobilization of the chemokine on the matrix via the biotin-streptavidin interaction. Next, the cells were added to the chemokine-matrix and incubated for 20 min at RT. The cells that did not bind to the matrix were removed by washing the matrix with PBS in a sterile 40um nylon filter (BD Falcon™ Cell Strainer). The flow through cells were stained with antibodies (Table 2), analysed by flow cytometry and compared with cells from peripheral blood that had not been incubated with the chemokine-matrix.

### Results and Discussion

### Diabetes Mellitus (DM)

### 1. Flow cytometric analysis of peripheral blood

White blood cells from DM patients were analysed with flow cytometry. About 80% of the monocytes are shown to express the chemokine receptor CCR2 (Fig 14) based upon flow cytometry data and binding by an anti-CCR2 antibody. CCR2 and its ligand MCP1 (CCL2) are important for monocyte migration and infiltration into the inflamed tissue. CCR2 is expressed on the majority of the monocytes in both diabetes patients and healthy controls. The expression of the CCR2 receptor on monocytes is not increased in diabetes patients; however, the monocytes in diabetes are potentially different in their pro-inflammatory profile with regards to other mediators. Furthermore, the ligand MCP-1 is secreted by inflamed and damaged tissue, such as the pancreas in diabetes, and will attract CCR2 expressing monocytes. In healthy individuals, this signal is absent and the monocytes will not migrate into the tissue even though they express CCR2. Thus, migration of pro-inflammatory CCR2 expressing monocytes is rather regulated by the level of the ligand MCP-1 than the level of CCR2 expression.

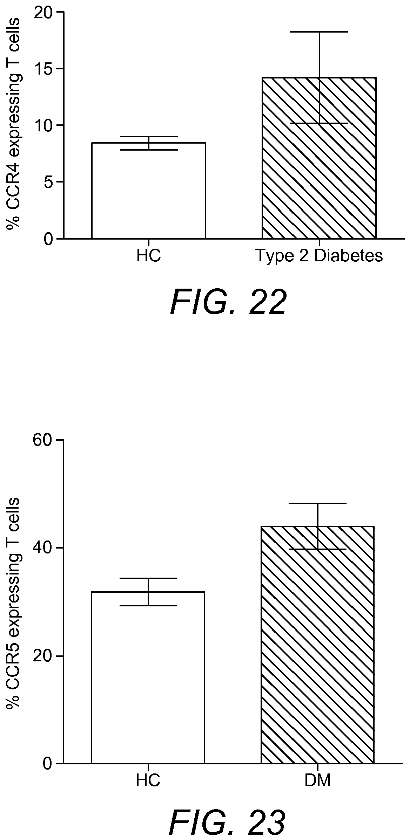

Figure 22 shows an increased frequency of CCR4 expressing T cells in four patients with type 2 Diabetes compared to healthy controls. Figure 23 shows an increased frequency of CCR5 expressing T cells in four patients with type 2 Diabetes compared to healthy controls. These cells may be depleted using suitable chemokines such as CCL17 (TARC) and CCL22 (MDC), which bind CCR4 only and CCL5 (RANTES) which binds CCR5.

### 2. Chemokine binding test

In accordance with the CCR2 expression, all the monocytes binds to biotinylated MCP1 (bMCP1) (Fig 15).

### 3. Cell depletion by matrix conjugated with biotinylated chemokine

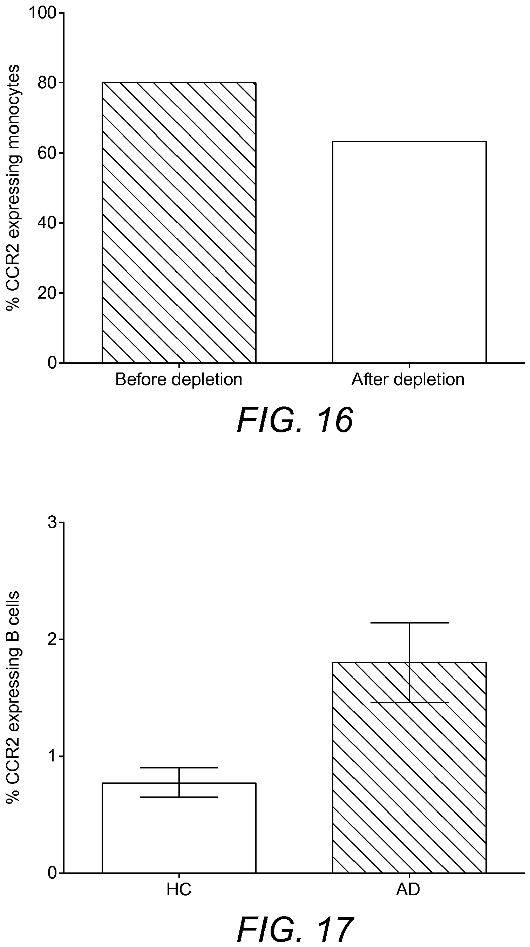

21% of the CCR2 expressing monocytes were efficiently depleted with bMCP1-conjugated Sepharose Streptavidin Matrix. Before depletion there were 80% CCR2 expressing monocytes and after depletion 63% (Fig 16).

We conclude that monocytes in DM express CCR2 and bind the ligand bMCP1. Furthermore, 20% of the CCR2 expressing monocytes can be removed with Sepharose Streptavidin matrix conjugated with bMCP1.

### EXAMPLE 11 - Diagnosis and treatment of Adiposis Dolorosa, reference/comparison example

### Materials and methods

### 1. Flow cytometric analysis of peripheral blood

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH₄Cl, pH 7.5) for 15 min at 37°C. The cells were washed in PBS with 2% Bovine Growth Serum, incubated with 10% human serum for 15min at room temperature (RT) and stained with antibodies (Table 3) at 4°C for 30min. The cells were analysed by flow cytometry on a FACS Canto flow cytometer with the FACSDiva software (BD Biosciences).

**Table 3. List of antibodies for flow cytometric analysis.**

| **Antibody** | **Fluorophore** | **Supplier** |
|---|---|---|
| CD14 | FITC | Beckman Coulter |
| Streptavidin | PE, APC | Biolegend |
| CCR2 | PerCP Cy5.5 | Biolegend |
| CD16 | PE Cy7 | BD Biosciences |
| HLADR | APC Cy7 | Biolegend |
| CD3 | V450 | BD Biosciences |
| CD19 | V500 | BD Biosciences |

### 2. Chemokine Binding test

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH4CI, pH 7.5) for 15 min at 37°C. The cells were washed in PBS with 2% Bovine Growth Serum, incubated with 10% human serum 15min at room temperature (RT) and stained with cell specific antibodies together with biotinylated chemokine (1µM) or the corresponding chemokine receptor antibody at 4°C for 30min (Table 3). The biotinylated chemokine was detected via the interaction between biotin and a fluorophore conjugated Streptavidin. The samples were analysed by flow cytometry on a FACS Canto flow cytometer with the FACSDiva software (BD Biosciences).

### 3. Cell depletion by matrix conjugated with biotinylated chemokine

Cells were prepared from peripheral blood (section 1). 1 mL Sepharose BigBeads matrix conjugated with 0.4mg/mL Streptavidin (GE Healthcare) was washed in 50 mL PBS and added to a 5 mL polystyrene tube (BD Falcon™). Biotinylated chemokine (1µM) was added to the tube and incubated for 20 min at RT to enable immobilization of the chemokine on the matrix via the biotin-streptavidin interaction. Next, the cells were added to the chemokine-matrix and incubated for 20 min at RT. The cells that did not bind to the matrix were removed by washing the matrix with PBS in a sterile 40um nylon filter (BD Falcon™ Cell Strainer). The flow through cells were stained with antibodies (Table 3), analysed by flow cytometry and compared with cells from peripheral blood that had not been incubated with the chemokine-matrix.

### RESULTS AND DISCUSSION

### 1. Flow cytometric analysis of peripheral blood

White blood cells from AD patients was analysed with flow cytometry. The patients exhibited two fold increased numbers of circulating CCR2 expressing B cells (mean of 1.8% compared to approximately 0.7% in healthy blood) (Fig 17).

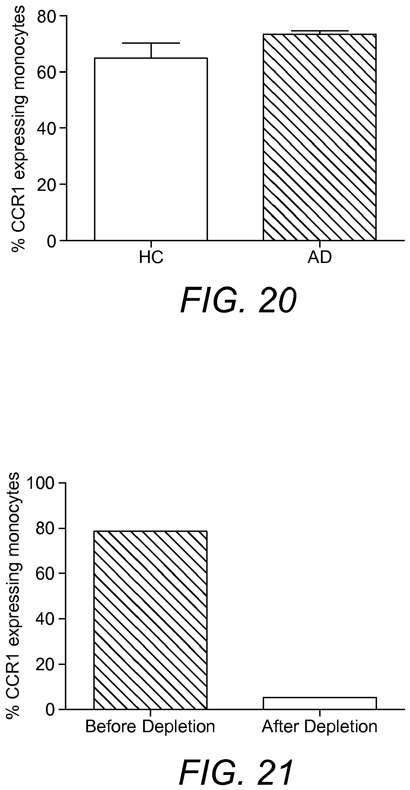

Figure 20 shows an increased frequency of CCR1 expressing monocytes in 4 patients with Addipois dolorosa compared to healthy controls.

### 2. Chemokine Binding test

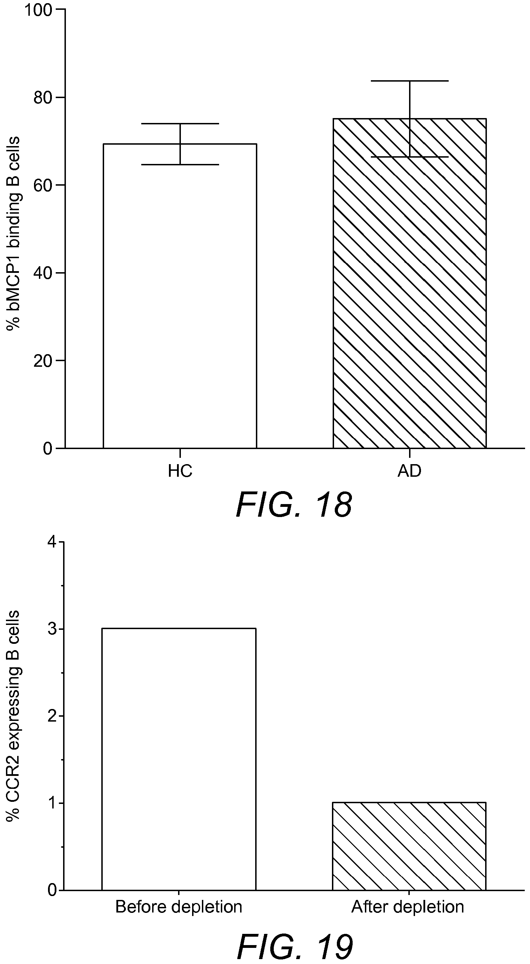

CCR2 binds to the chemokine MCP1 (CCL2) and is important for cell recruitment to inflammation sites. 65% of the B cells bind to the biotinylated MCP1 (bMCP1) (Fig 18).

### 3. Cell depletion by matrix conjugated with biotinylated chemokine

67% of the CCR2 expressing B cells were efficiently depleted with bMCP1-conjugated Sepharose Streptavidin Matrix. Before depletion there were 3% CCR2 expressing B cells and after depletion 1% (Fig 19).

We conclude that B cells in AD express CCR2 and bind the ligand bMCP1. Furthermore, 67% of the CCR2 expressing B cells can be removed with Sepharose Streptavidin matrix conjugated with bMCP1.

Figure 21 shows depletion of CCR1 expressing moncoytes with SepharoseStrepavidin-matrix- bRANTES. Blood cells from a healthy control were incubated with biotinylated chemokine-Sepharose Streptavidin-matrix. Unbound cells were retrieved by washing the matrix. The cells (After Depletion) were then analysed with flow cytometry and compared with cells that had not been incubated with bchemokine-matrix (Before Depletion). Thus, monocytes expressing CCR1 are upregulated in AD. These cells can be effectively depleted using a suitable chemokine, in this case biotinylated CCL5 (RANTES).

## Claims

1. A binding reagent capable of specifically binding to the chemokine receptor CCR2 for use in the treatment of a diabetes condition, wherein the binding reagent is immobilized on a solid support contained within an apheresis column, to which is applied peripheral blood from a patient thus removing CCR2 expressing cells from the peripheral blood of the patient, and wherein the binding reagent is MCP-1.

2. The binding reagent for use according to claim 1, wherein:
(i) the cells are CCR2 expressing monocytes and where diabetes is treated; and/or
(ii) the patient has been selected for treatment on the basis of detecting an increase in the level of CCR2 expressing cells, levels of expression of CCR2 and/or levels of cells with high expression of CCR2 in a sample obtained from the patient;
and/or
(iii) 20-50% of the patient's blood is applied to the column in a single treatment.

3. A modified MCP-1 chemokine for use according to any one of claims 1-2, comprising the amino acid sequence set forth as SEQ ID NO: 1, SEQ ID NO: 2 or SEQ ID NO: 3 in which one or more of the following modifications have been made:
a) the glutamine residue at position 1 of SEQ ID NO: 2 has been replaced with pyroglutamine
b) the C terminus is produced as an amide derivative
c) the lysine residue at position 98 of SEQ ID NO: 1 or position 75 of SEQ ID NO:2 or position 67 of SEQ ID NO: 3, which may be replaced by a suitable alternative amino acid such as diaminopropionic acid or ornithine, is biotinylated, optionally via a spacer group, in order to permit immobilization of the chemokine on a solid support
d) the methionine residue at position 87 of SEQ ID NO: 1 or position 64 of SEQ ID NO: 2 or position 56 of SEQ ID NO: 3 has been replaced with norleucine.

4. The modified MCP-1 chemokine for use according to claim 3 wherein:
(i) the lysine residue at position 98 of SEQ ID NO: 1 or position 75 of SEQ ID NO:2 or position 67 of SEQ ID NO: 3 is biotinylated via a polyethylene glycol (PEG) spacer group, in order to permit immobilization of the chemokine on a solid support optionally whereinthe PEG spacer is 3,6-dioxo aminooctanoic acid;
and/or
(ii) the modified MCP-1 chemokine has the structure shown in any one of figures 7 to 9;
and/or
(iii) the modified MCP-1 chemokine is an agonist or an antagonist of CCR2 activity.

5. A modified MCP-1 chemokine for use according to any one of claims 1-2, comprising the amino acid sequence of SEQ ID NO: 9 or SEQ ID NO: 11.

## Patentansprüche

1. Bindungsreagenz, welches zur Verwendung bei der Behandlung von einem Diabeteszustand an den Chemokinrezeptor CCR2 spezifisch binden kann, wobei das Bindungsreagenz auf einem innerhalb einer Apheresesäule enthaltenen festen Träger immobilisiert wird, auf welche peripheres Blut von einem Patienten aufgetragen wird, wodurch CCR2 exprimierende Zellen aus dem peripheren Blut des Patienten entfernt werden, und wobei das Bindungsreagenz MCP-1 ist.

2. Bindungsreagenz zur Verwendung nach Anspruch 1, wobei:
(i) die Zellen CCR2 exprimierende Monozytten sind, und wobei Diabetes behandelt wird;
und/oder
(ii) der Patient auf Grundlage eines Detektierens einer Erhöhung des Pegels von CCR2 exprimierenden Zellen, der Pegel der Expression von CCR2 und/oder der Pegel von Zellen mit hoher Expression von CCR2 in einer von dem Patienten erhaltenen Probe für Behandlung ausgewählt worden ist;
und/oder
(iii) 20-50% des Bluts des Patienten in einer einzigen Behandlung auf die Säule aufgetragen wird.

3. Modifiziertes MCP-1-Chemokin zur Verwendung nach einem der Ansprüche 1-2, umfassend die Aminosäuresequenz mit SEQ ID NO: 1, SEQ ID NO: 2 oder SEQ ID NO: 3, worin eine oder mehrere der folgenden Modifikationen ausgeführt worden sind:
a) der Glutamin-Rest an der Position 1 von SEQ ID NO: 2 ist durch Pyroglutamin ersetzt worden
b) das C-terminale Ende ist als ein Amidderivat hergestellt
c) der Lysin-Rest an der Position 98 mit SEQ ID NO: 1 oder an der Position 75 mit SEQ ID NO:2 oder an der Position 67 mit SEQ ID NO: 3, welcher durch eine geeignete alternative Aminosäure, wie beispielsweise Diaminopropions-äure oder Ornithin, ersetzt sein kann, ist biotinyliert, wahlweise durch eine Spacergruppe, um einer Immobilisierung des Chemokins auf einem festen Träger zu gestatten
d) der Methionin-Rest an der Position 87 mit SEQ ID NO: 1 oder an der Position 64 mit SEQ ID NO: 2 oder an der Position 56 mit SEQ ID NO: 3 ist durch Norleucin ersetzt worden.

4. Modifiziertes MCP-1-Chemokin zur Verwendung nach Anspruch 3, wobei:
(i) der Lysin-Rest an der Position 98 mit SEQ ID NO: 1 oder an der Position 75 mit SEQ ID NO:2 oder an der Position 67 mit SEQ ID NO: 3 durch eine Polyethylen-Glycol- (PEG)-Spacergruppe biotinyliert ist, um einer Immobilisierung des Chemokins auf einem festen Träger zu gestatten, wahlweise wobei der PEG-Spacer 3,6-Dioxoaminooctansäure ist;
und/oder
(ii) das modifizierte MCP-1-Chemokin die Struktur wie in irgendeiner der Figuren 7 bis 9 gezeigt aufweist;
und/oder
(iii) das modifizierte MCP-1-Chemokin ein Agonist oder ein Antagonist mit CCR2-Aktivität ist.

5. Modifiziertes MCP-1-Chemokin zur Verwendung nach einem der Ansprüche 1-2, umfassend die Aminosäuresequenz mit SEQ ID NO: 9 oder SEQ ID NO: 11.

## Revendications

1. Réactif de liaison capable de se lier spécifiquement à un récepteur de chimiokine CCR2 à utiliser dans le traitement d'une condition de diabète, ledit réactif de liaison étant immobilisé sur un support solide contenu dans une colonne d'aphérèse, à laquelle est appliqué le sang périphérique d'un patient, éliminant ainsi des cellules exprimant CCR2 du sang périphérique du patient, le réactif de liaison étant MCP-1.

2. Réactif de liaison pour l'usage selon la revendication 1, dans lequel :
(i) les cellules sont des monocytes exprimant CCR2 où le diabète est traité ;
et / ou
(ii)le patient a été sélectionné pour le traitement sur la base de la détection d'une augmentation du niveau des cellules exprimant CCR2, des niveaux d'expression de CCR2 et/ou des niveaux de cellules avec une expression élevée de CCR2 dans un échantillon obtenu à partir du patient ;
et / ou
(iii) 20-50% du sang du patient est appliqué sur la colonne dans un seul traitement.

3. Chimiokine MCP-1 modifiée pour l'usage selon l'une quelconque des revendications 1 à 2, comprenant la séquence d'acides aminés présentée comme SEQ ID NO: 1, SEQ ID NO: 2 ou SEQ ID NO: 3 dans laquelle l'une ou plusieurs des modifications suivantes ont été réalisées :
a) le résidu de lysine à la position 1 de SEQ ID NO: 2 a été remplacé par pyroglutamine
b) l'extrémité C est produite sous la forme d'un dérivé amide
c) le résidu de lysine à la position 98 de SEQ ID NO: 1 ou position 75 de SEQ ID NO:2 ou la position 67 de SEQ ID NO: 3, qui peut être remplacée par un acide aminé alternatif approprié tel que l'acide diaminopropionique ou l'ornithine, est biotinylée, éventuellement par un groupe espaceur, pour permettre l'immobilisation de la chimiokine sur un support solide
d) le résidu de méthionine à la position 87 de SEQ ID NO: 1 ou à la position 64 of SEQ ID NO: 2 ou à la position 56 of SEQ ID NO: 3 a été remplacé par norleucine.

4. Chimiokine MCP-1 modifiée pour l'usage selon la revendication 3, dans laquelle :
(i) le résidu de lysine à la position 98 de SEQ ID NO: 1 ou position 75 de SEQ ID NO:2 ou la position 67 de SEQ ID NO: 3 est biotinylé via un groupe d'espaceur en polyéthylène glycol (PEG), afin de permettre l'immobilisation de la chimiokine sur un support solide, éventuellement où l'espaceur PEG est l'acide 3,6-dioxo aminooctanoïque ;
et / ou
(ii) la chimiokine MCP-1 modifiée ayant la structure montrée dans l'une quelconque des figures 7 à 9 ;
et / ou
(iii) la chimiokine MCP-1 modifiée est un agoniste ou un antagoniste d'activité de CCR2.

5. Chimiokine MCP-1 modifiée pour l'usage selon l'une quelconque des revendications 1 à 2, comprenant la séquence d'acides aminés de SEQ ID NO: 9 ou SEQ ID NO: 11.
